# EUROPEAN PATENT SPECIFICATION

(11) **EP 3 638 774 B1**
(45) Date of publication and mention of the grant of the patent: **07.08.2024**
(21) Application number: 18736809.7
(22) Date of filing: 14.06.2018
(51) Int. Cl.: C12N 5/071

(54) **METHODS FOR PURIFYING ENDODERM AND PANCREATIC ENDODERM CELLS DERIVED FROM HUMAN EMBRYONIC STEM CELLS**
VERFAHREN ZUR REINIGUNG VON ENDODERMEN UND PANKREATISCHEN ENDODERMEN ZELLEN AUS MENSCHLICHEN EMBRYONALEN STAMMZELLEN
PROCÉDÉS DE PURIFICATION D'ENDODERME ET DE CELLULES D'ENDODERME PANCRÉATIQUE DÉRIVÉES DE CELLULES SOUCHES EMBRYONNAIRES HUMAINES

(30) Priority: 14.06.2017 LU 100320
(43) Date of publication of application: 22.04.2020
(73) Proprietor: Helmholtz Zentrum München - Deutsches Forschungszentrum für Gesundheit und Umwelt (GmbH), 85764 Neuherberg (DE); Miltenyi Biotec B.V. & Co. KG, 51429 Bergisch Gladbach (DE)
(72) Inventor: LICKERT, Heiko, 80469 München (DE); MAHADALKAR, Pallavi, 421203 Maharashtra (IN); KNÖBEL, Sebastian Mathias, 50999 Köln (DE)
(74) Representative: Schiweck Weinzierl Koch Patentanwälte Partnerschaft mbB
(86) International application number: PCT/EP2018/065779
(87) International publication number: WO 2018/229179

(56) References cited:
- WO-A1-01/23554
- WO-A1-2009/131568
- WO-A1-2014/030166
- WO-A2-2012/070014
- MAHADDALKAR PALLAVI U ET AL: "Generation of pancreatic [beta] cells from CD177anterior definitive endoderm", NATURE BIOTECHNOLOGY, NATURE PUBLISHING GROUP US, NEW YORK, vol. 38, no. 9, 27 April 2020 (2020-04-27), pages 1061 - 1072, XP037237846, ISSN: 1087-0156, [retrieved on 20200427], DOI: 10.1038/S41587-020-0492-5
- XIN CHENG ET AL: "Self-Renewing Endodermal Progenitor Lines Generated from Human Pluripotent Stem Cells", CELL STEM CELL, vol. 10, no. 4, 1 April 2012 (2012-04-01), AMSTERDAM, NL, pages 371 - 384, XP055305944, ISSN: 1934-5909, DOI: 10.1016/j.stem.2012.02.024

## Description

### BACKGROUND

Beta-cell destruction in the pancreatic islets by an autoimmune reaction is the cause of diabetes mellitus type I, but also in diabetes mellitus type II β-cell destruction may happen in later stages of the disease, although mediated by other mechanisms. However, patients with these diseases, especially diabetes mellitus type I, require exogenous insulin delivery, but choosing the right insulin dosage remains a challenge and may lead to poor glycemic control.

The ability of regeneration of pancreatic islets is very limited, so transplantation of islet cells can lead to an improved glucose tolerance and control. However, islet cell transplantation suffers from different limitations including reliance on donor organs, challenges with isolation of islets from the pancreas and life-long use of immunosuppressive drugs. Pluripotent stem cells pose a possible solution for the shortage of donor islet cells.

Several methods for the differentiation of pluripotent or embryonic stem cells to pancreatic progenitor cells or β-cells have been described in prior art and are cited here for reference only. Examples are Rezania et al., 2014, Pagliuca et al., 2014, D'Amour et al., 2006 or Kroon et al., 2008. While all these methods lead to a significant ratio of β-cells, the β-cells generated by these methods are immature and may also contain some undifferentiated pluripotent stem cells that can pose a risk for teratoma formation. WO 2012/070014 discloses a method for identifying pancreatic progenitor cells differentiated from pluripotent stem cells by determining the expression of different cell markers.

Therefore, it is important to enrich subpopulations of cells during the differentiation that lead to a larger portion of pancreatic progenitor cells. During the development of the pancreas and pancreatic islets the cells go through various intermediates. One important intermediate is the stage of the anterior definitive endoderm (ADE). Here it is possible to differentiate between already specified subpopulations developing into the pancreatic lineage or other lineages like e.g. liver. The patent applications US 2009/0263896 A1 and WO2016/170069 A1 disclose several markers for the enrichment of pancreatic endoderm cells. However, those patent application sort for subpopulations at later stages of differentiation towards pancreatic beta-cells. Those cells upregulate cyclin-dependent kinase inhibitors and lose the potential for expanding large quantities of cells for cell-replacement therapy. Sorting pancreatic progenitors at the ADE stage, when cells are still highly proliferative, would overcome the expansion problem and allow scalable production of large quantities of pancreatic progenitor cells from pluripotent stem cells.

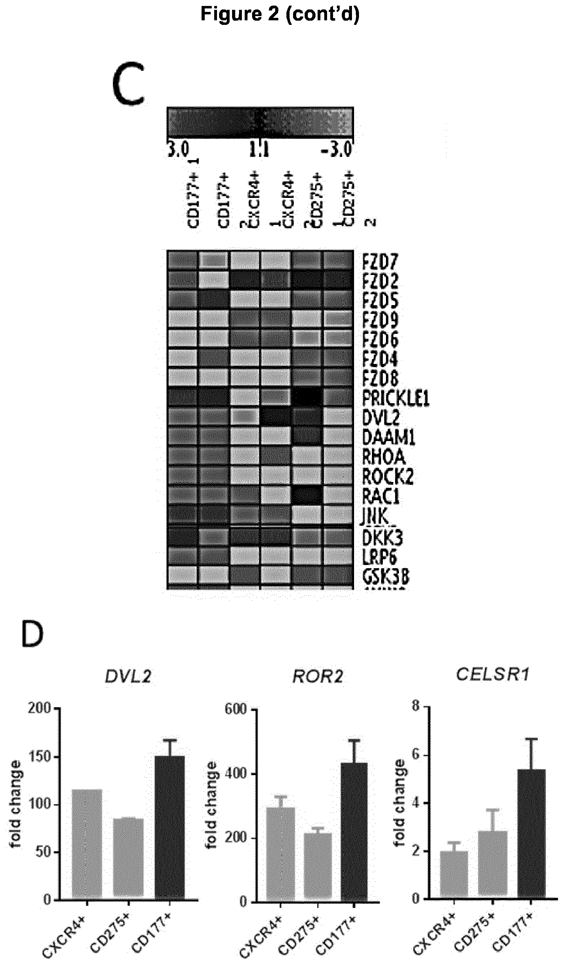

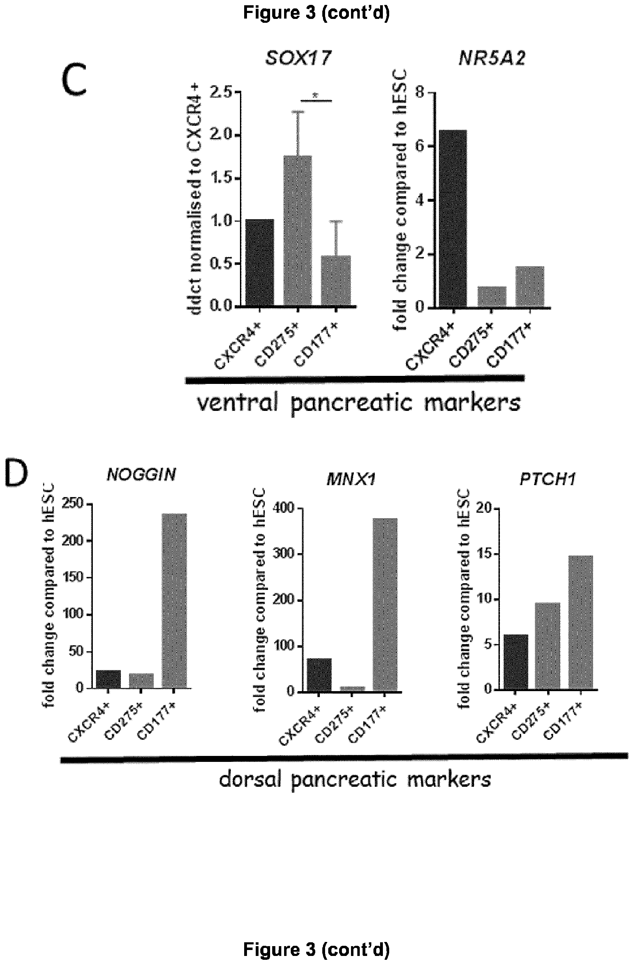

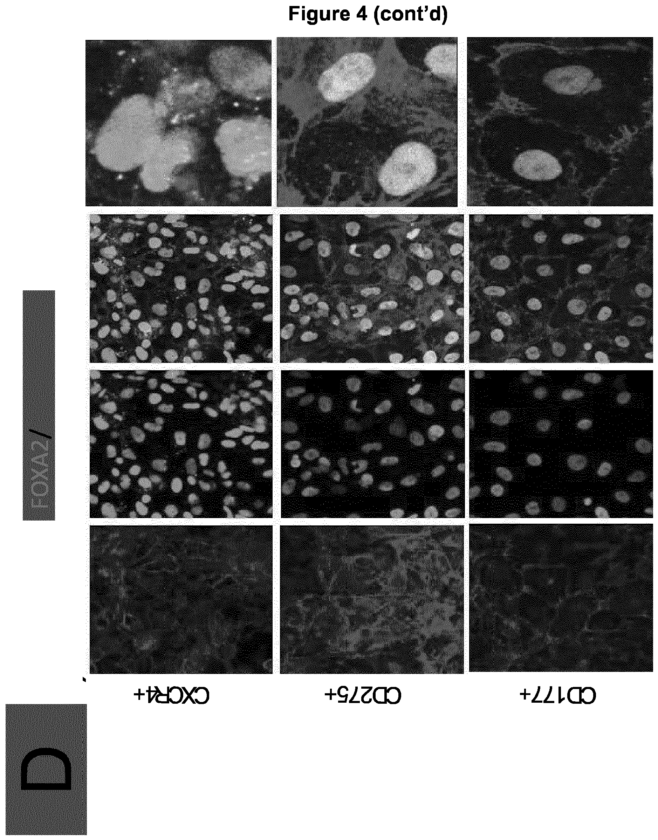

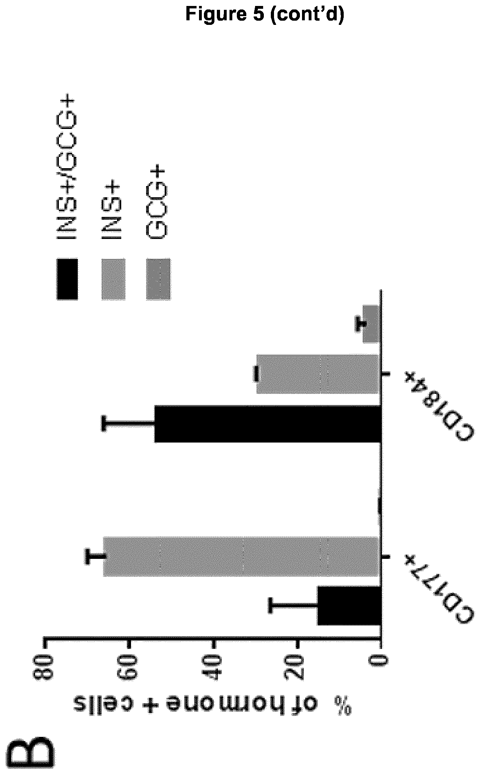

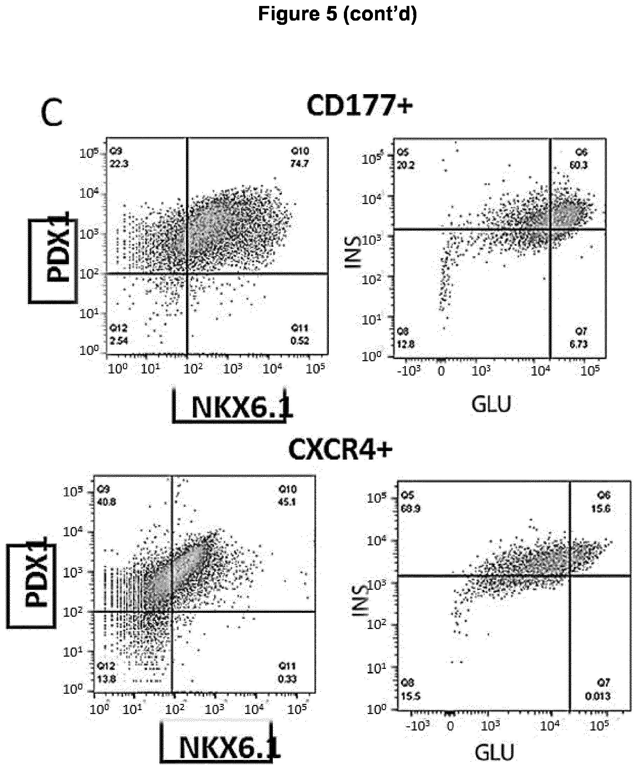

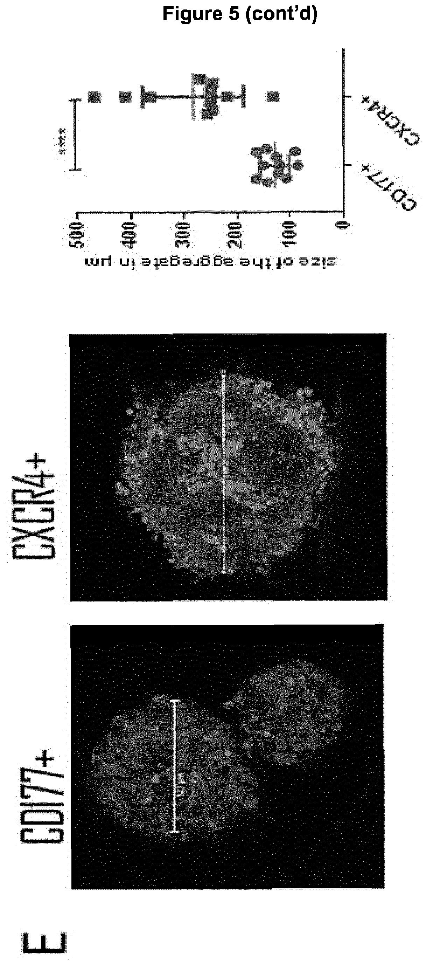

However, there is still a need in new reliable and preferably improved markers for definitive endoderm subpopulations with a pancreatic fate at an earlier stage of differentiation, thereby ideally improving the selection of specified pancreatic progenitor cells out of a heterogeneous cell population, which advantageously still has the ability to proliferate during the following manufacturing process. Accordingly the technical problem underlying the present application is to comply with this need. The inventors of the present invention surprisingly found that the cell surface markers CD51 and/or CD177 can be used to enrich future pancreatic progenitor cells at the stage of definitive endoderm, whereas the cell surface marker CD275 can be used to enrich future cells of the liver lineage or for depletion of such cells. Depletion of cells of the liver lineage can be used to remove cells not developing into pancreatic progenitor cells, thereby enriching cells capable of developing into pancreatic progenitor cells. In addition, teratoma forming cells can be sorted out and CD177 and CD275 can serve as tool to assess differentiation process in different cell lines and different protocols. CD177 subpopulations additionally show increased WNT/planar cell polarity (PCP) signaling. The CD177+ cells show translocation of the β-catenin to the membrane, thus promoting pancreatic fate over hepatic fate (Examples 2 and 4, Fig. 2C-E, Fig.4D). CD177+ cells efficiently give rise to dorsal pancreas (Examples 3 and 7, Fig. 3C,D), generate tighter clusters and have tissue polarity (Example 5, Fig. 5E). They show higher expression of pancreatic transcription factors like PDX1 or NKX6.1 (Examples 5 and 9, Fig. 5C) and generate monohormonal cells (Examples 5 and 9, Fig. 5B). In addition, as shown in Examples 2, 6 and 7, CD177-positive cells gives rise to cell populations with a higher probability to become pancreatic progenitor cells than CXCR4-positive cells (CD184) that are known in prior art.

### SUMMARY OF THE INVENTION

Embodiments described herein relate to methods for purifying pancreatic progenitor cells derived from pluripotent stem cells, induced pluripotent stem cells and/or embryonic stem cells and compositions comprising said purified pancreatic progenitor cells. The invention is set out in the appended set of claims.

Accordingly, the present invention relates to a method of purifying a pancreatic progenitor cell comprising:
(a) exposing a population of cells derived from pluripotent stem cells, induced pluripotent stem cells and/or embryonic stem cells comprising a pancreatic progenitor cell, to a ligand which binds to a cell surface marker on the pancreatic progenitor cell, wherein the cell surface marker is CD177 and
(b) separating the pancreatic progenitor cell from cells derived from pluripotent stem cells, induced pluripotent stem cells and/or embryonic stem cells which do not bind to the ligand, thereby purifying said pancreatic progenitor cell,
wherein the pancreatic progenitor cell is sorted at the stage of the definitive endoderm (DE), the anterior definitive endoderm (ADE), the posterior gut tube (PGT) and/or the ventral foregut (VFG), wherein the pancreatic progenitor cell preferably comprises CD177 on the cell surface.

Another advantage of the method of purifying a (specified) pancreatic progenitor cell according to the invention may be the enablement of a scalable production system. The early selection of (specified) pancreatic progenitor cells during the differentiation process, e.g. at the stage of the DE, the ADE or the PGT allows sorting out of small cell populations. Those cells are then expanded afterwards and material (including media, cytokines etc.) is saved. In contrast, prior art sorts at late stages of differentiation. At this point, already a lot of material has already been used. Thus, a big portion of this is wasted. The present invention therefore provides a more economic method for the manufacturing of cells of the pancreatic lineage.

The pancreatic progenitor cell which is purified in accordance with the method of the present invention preferably expresses FOXA2, SOX17, CER1 and/or CXCR4, more preferably CER1.

The method of the present invention comprises preferably a further step, i.e. the step of exposing said population of cells to a ligand which binds to CD51 and/or CD275.

Preferably, the ligand that is binding to CD51, CD177 and/or CD275 used for purifying pancreatic progenitor cells in accordance with the method of the present invention is an antibody or binding fragment thereof.

The antibody or antibody fragment that is binding to CD51, CD177 and/or CD275 used in accordance with the method of the present invention preferably is a monoclonal antibody or a polyclonal antibody.

Preferably, the ligand, which is binding to CDS1, CD177 and/or CD275 and used for purifying pancreatic progenitor cells in accordance with the method of the present invention, is conjugated with a label.

Preferably, the expression of the cell surface markers CD51, CD177 and/or CD275 is detected by quantitative polymerase chain reaction (qPCR) and/or flow cytometry in accordance with the method of purification of pancreatic progenitor cells described herein.

The separation step of the method according to the present invention preferably comprises affinity chromatography and/or flow cytometry.

The pancreatic progenitor cells purified in accordance with the method of the present invention preferably are human.

The ligand used in the method in accordance to the present invention or the composition of pancreatic progenitor cells described herein preferably is immobilized on a capture medium. The capture medium preferably comprises a bead and/or the ligand preferably is conjugated to a magnetic reagent.

The method of purifying pancreatic progenitor cells in accordance with the present invention preferably results in a cell composition comprising at least 50% (specified) pancreatic progenitor cells.

A composition purified according to the method of the invention comprises at least 50% pancreatic progenitor cells which are positive for CD177, wherein the pancreatic progenitor cell preferably comprises CD177 on the cell surface. Preferably the pancreatic progenitor cells of the composition described herein are bound to a ligand that binds CD177 on the pancreatic progenitor cells. The composition of pancreatic progenitor cells purified in accordance to the method of the present invention and/or the pancreatic progenitor cells of the composition bound to a ligand that binds CD177 on the pancreatic progenitor cells preferably are human.

Further, the present invention relates to the use of a ligand which binds to a cell surface marker on a pancreatic progenitor cell, wherein the cell surface marker is CD177, for purifying a pancreatic progenitor cell from a population of cells derived from pluripotent stem cells, induced pluripotent stem cells and/or embryonic stem cells, wherein the pancreatic progenitor cell is sorted at the stage of the definitive endoderm (DE), the anterior definitive endoderm (ADE), the posterior gut tube (PGT) and/or the ventral foregut (VFG), wherein the pancreatic progenitor cell preferably comprises CD177 on the cell surface.

It will be appreciated that the methods and compositions described above relate to cells cultured in vitro. However, the above-described in vitro differentiated cell compositions may be used for in vivo applications.

### BRIEF DESCRIPTIONS OF THE DRAWINGS

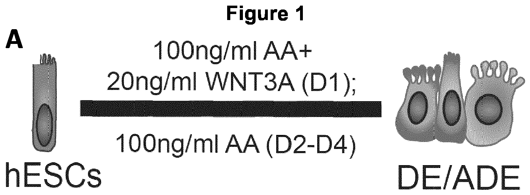

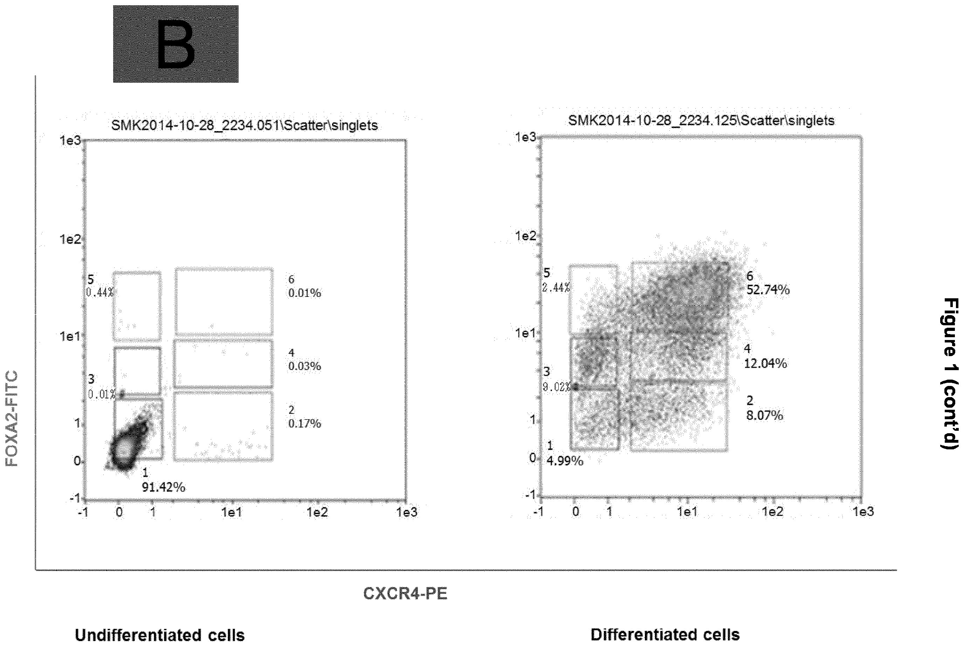

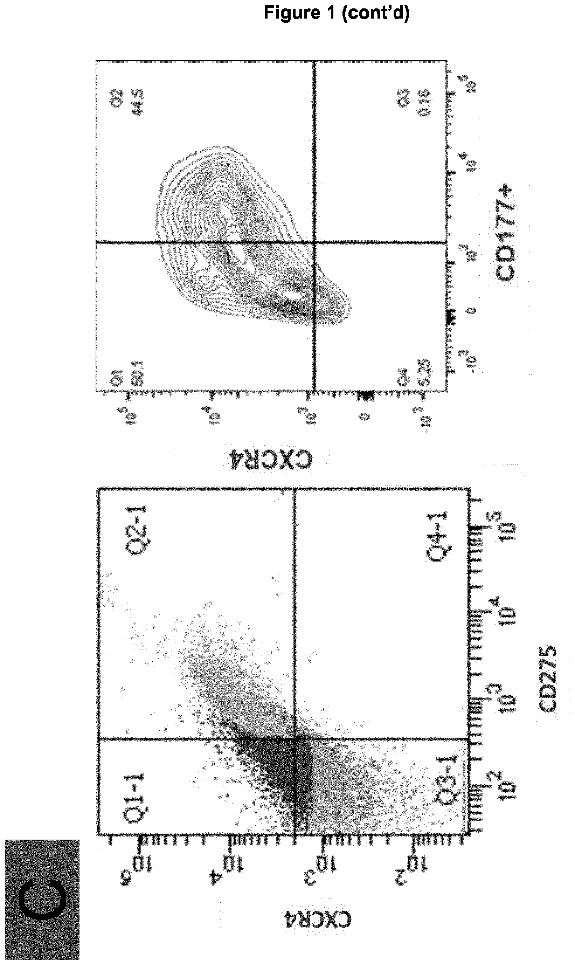

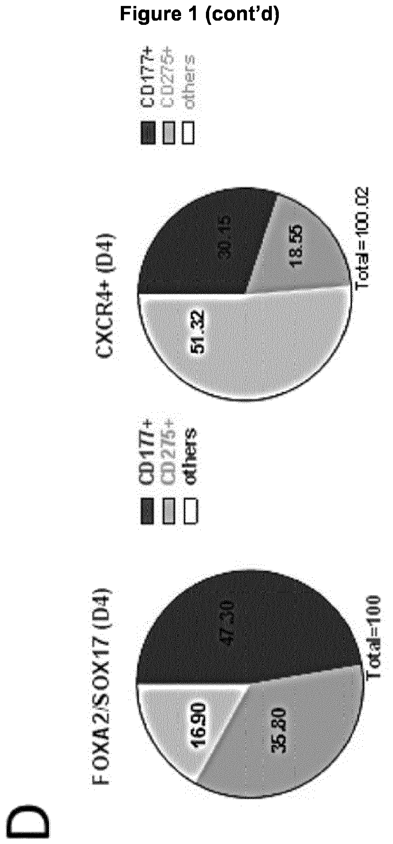

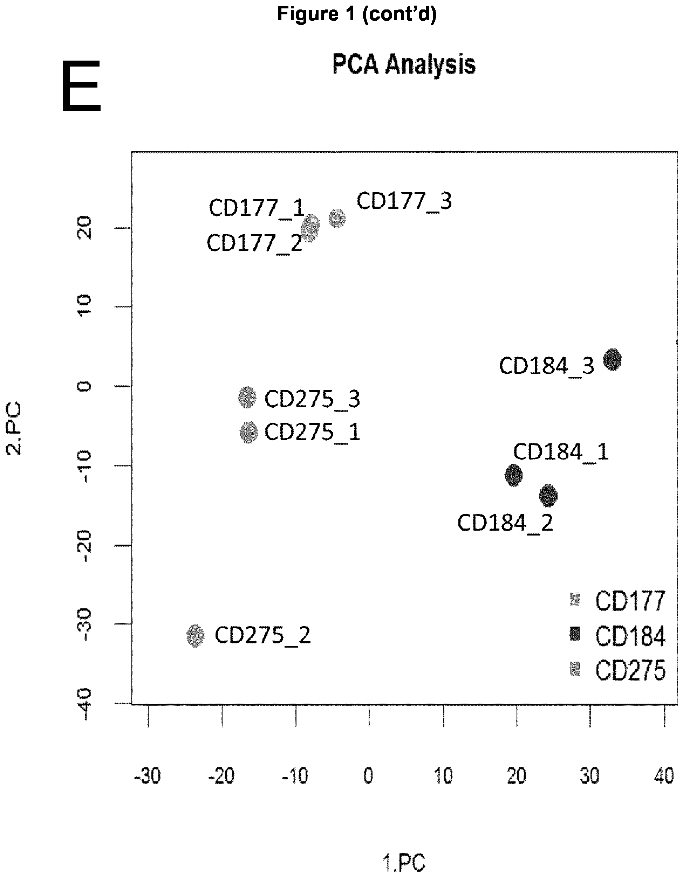

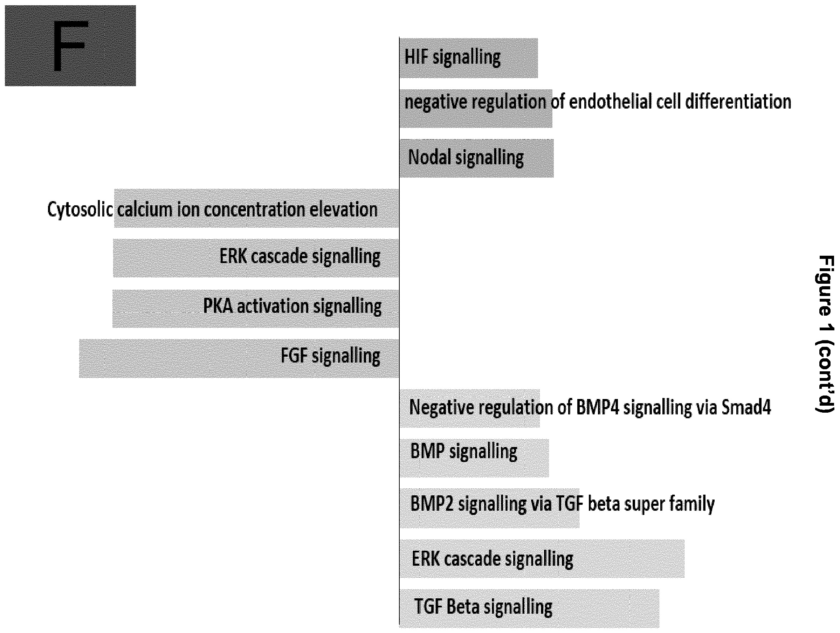

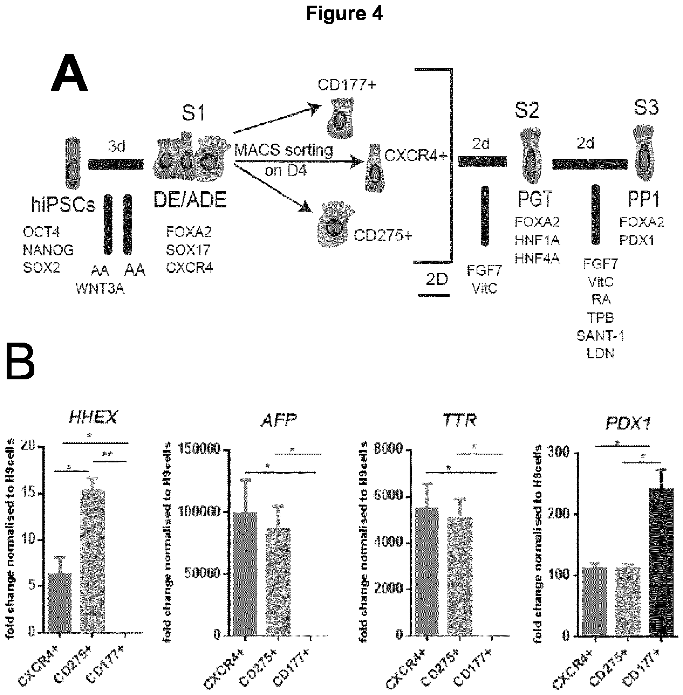

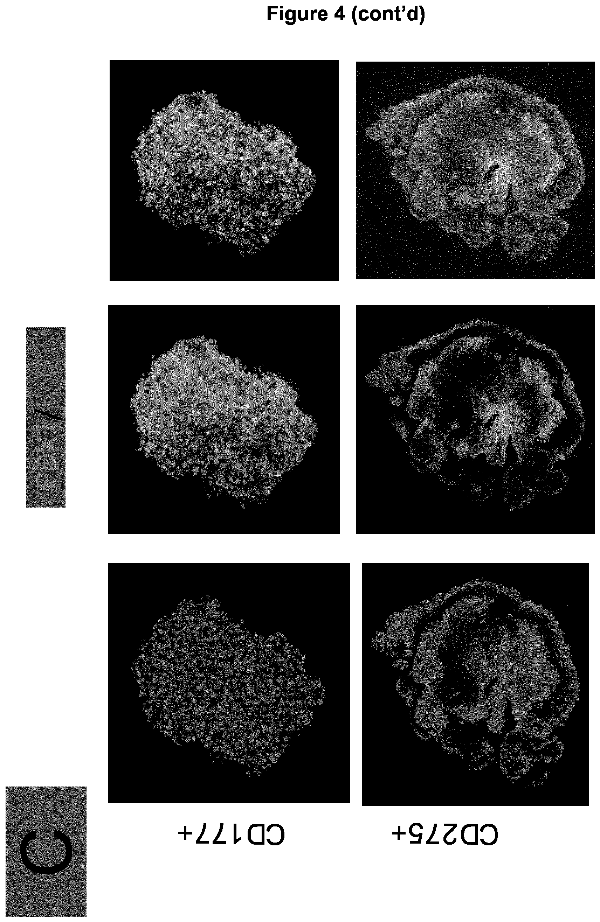

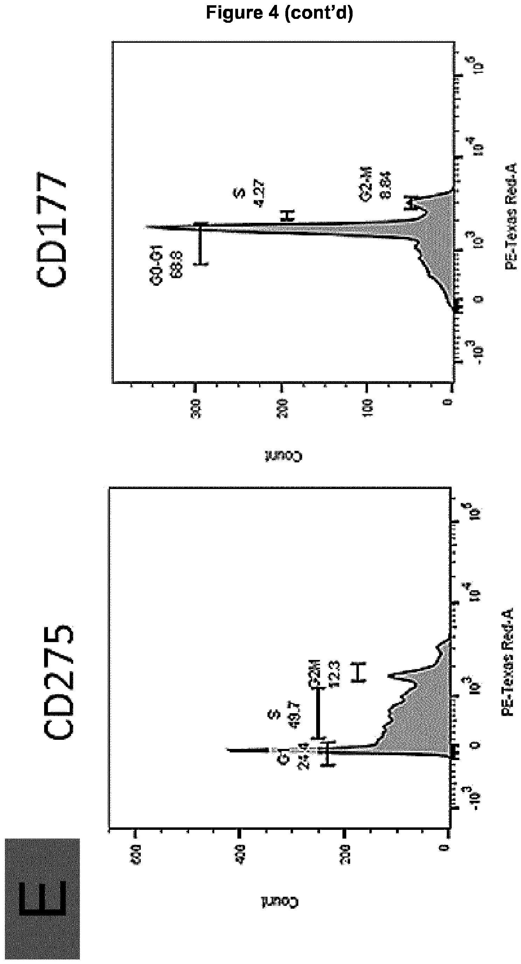

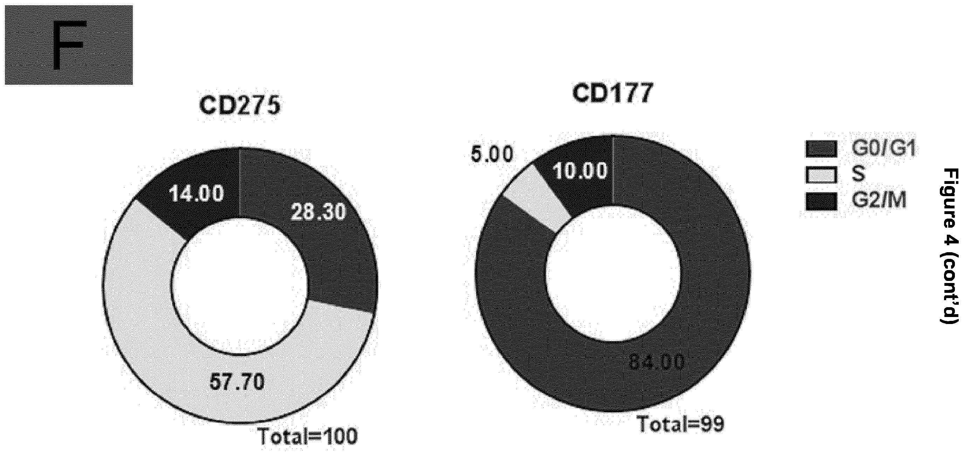

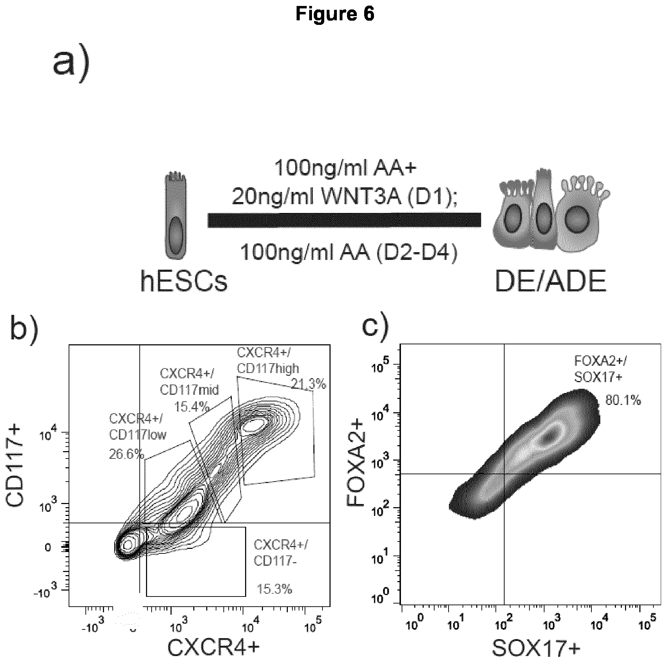

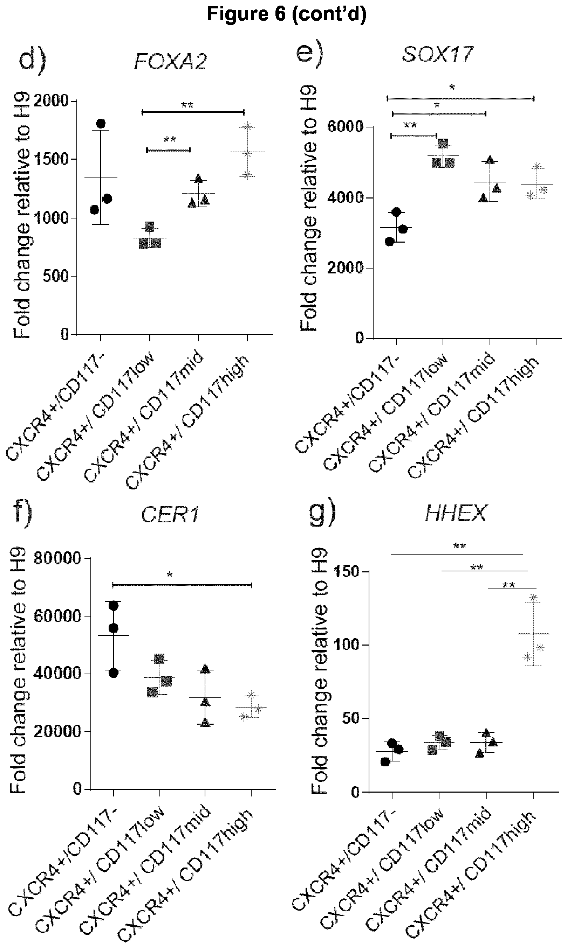

**Figure 1****: Screening set up for identification of novel surface marker antibodies that enrich for different definitive endoderm (DE).**
   A) Schematic representation of the differentiation of hESCs towards DE
   B) Setup for identification of novel surface markers that can enrich different DE subpopulations against known DE markers FOXA2 and CXCR4
   C-D) CD275 and CD177 enrich distinct DE subpopulations in the FOXA2/SOX17 and CXCR4+ DE E) PCA analysis of the sorted DE subpopulations at D4 indicating CD177 and CD275 DE is different from CXCR4 (CD184) DE
   F) Gene ontology analysis of GO terms arising from genes up-regulated >1.5 fold at day 4 after differentiation.

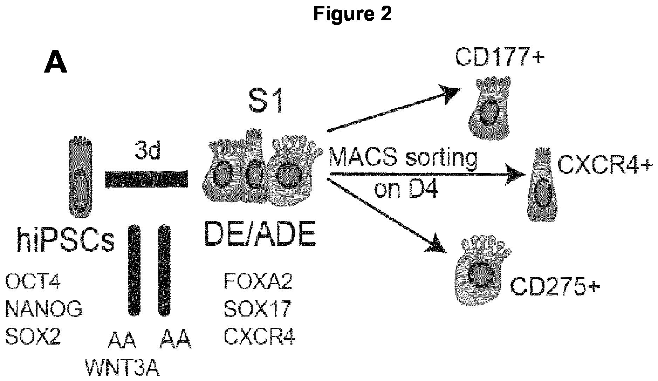

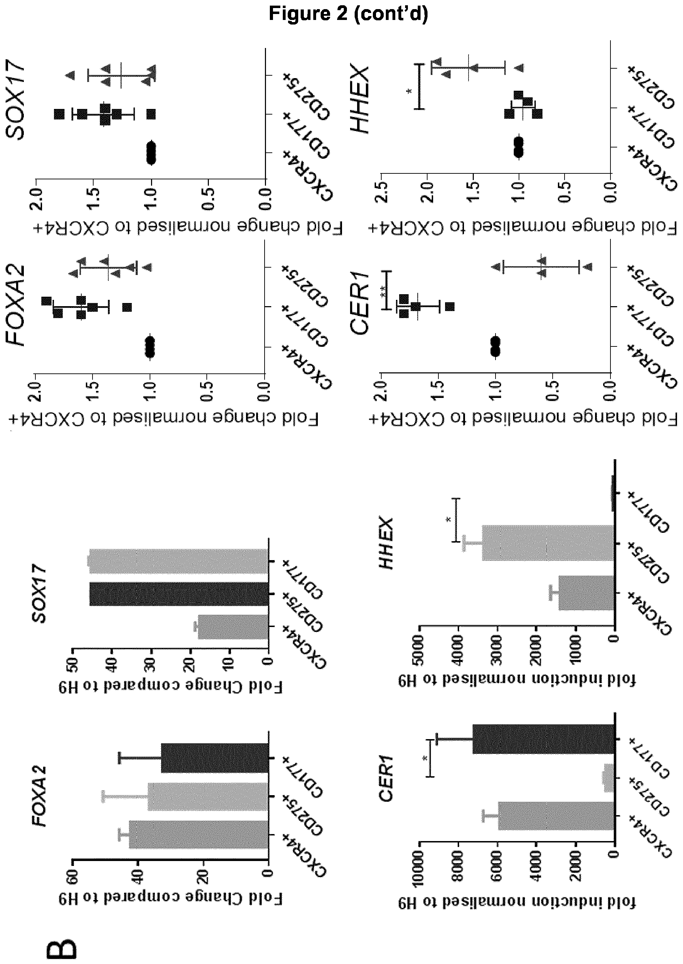

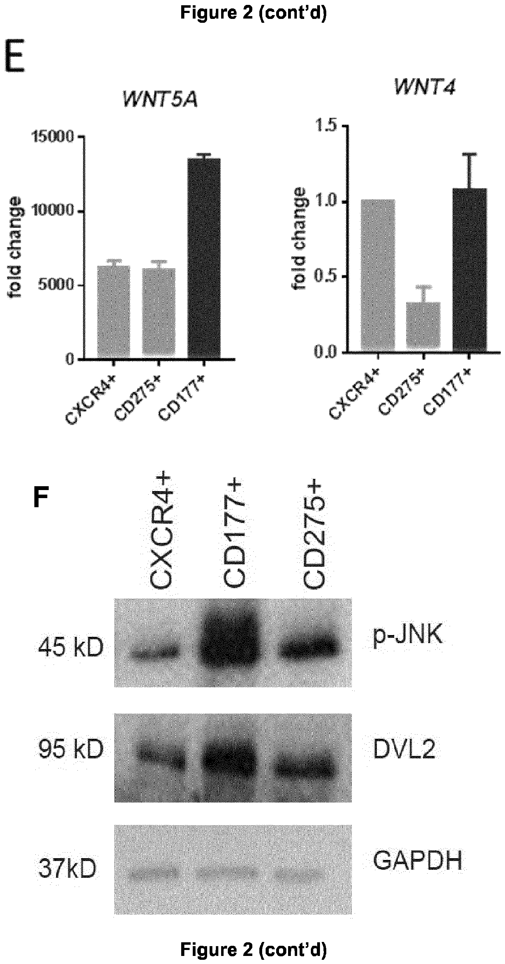

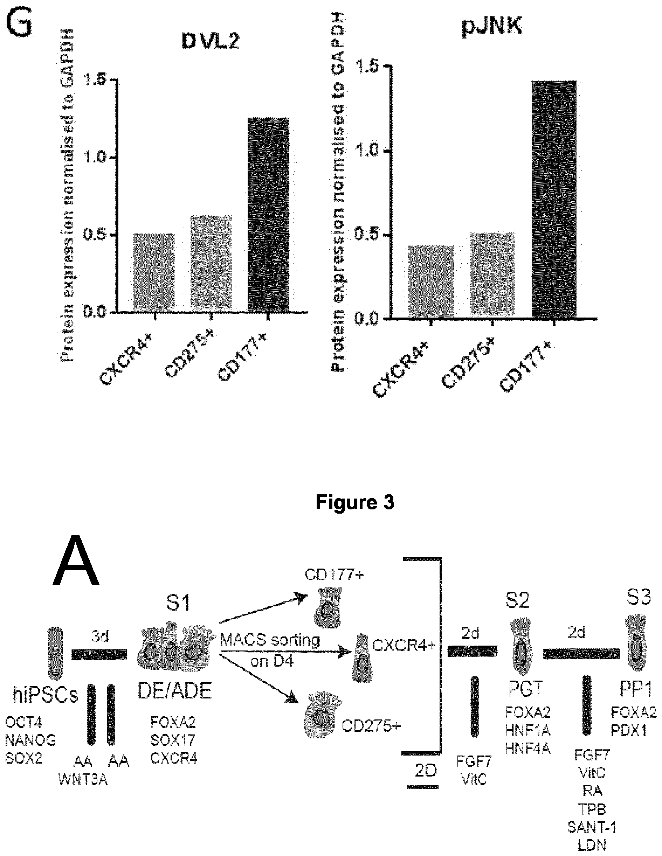

**Figure 2****: Non-canonical WNT/PCP pathway is differentially regulated in CD177+ subpopulation.**
   A) Schematic representation of the differentiation of hESCs towards DE and sorting using MACS
   B) mRNA quantification indicating similar expression of FOXA2 and SOX17 in all subpopulations but distinct profiles in terms of anterior definitive endoderm (ADE) markers CER1 and HHEX
   C) Microarray profiling showing upregulation of WNT/PCP target genes in CD177+ DE subpopulation compared to CXCR4 and CD275 subpopulations.
   D-E) qPCR analysis confirming upregulation of WNT/PCP target genes (DVL2, ROR2, CELSR1) and ligands (WNTSA and WNT4) in CD177+ DE.
   F-G) Western blot analysis showing phosphorylation WNT/PCP target genes p-JNK in CD177 compared to CD275 and CXCR4+ DE.

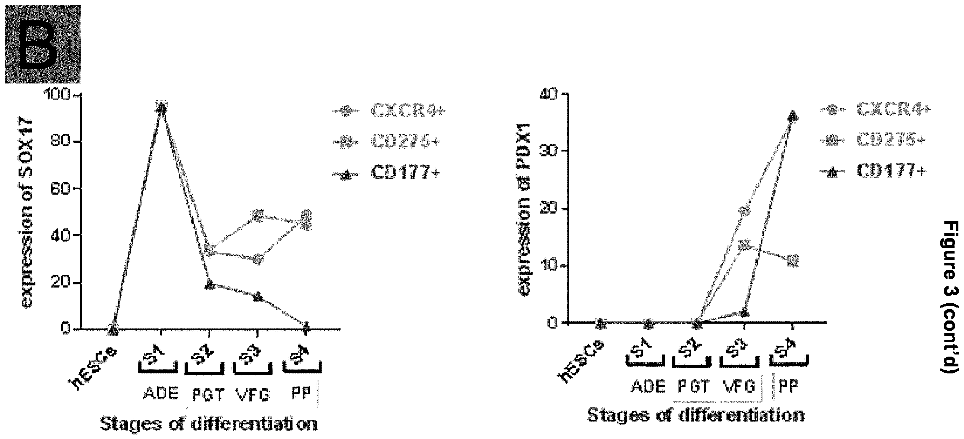

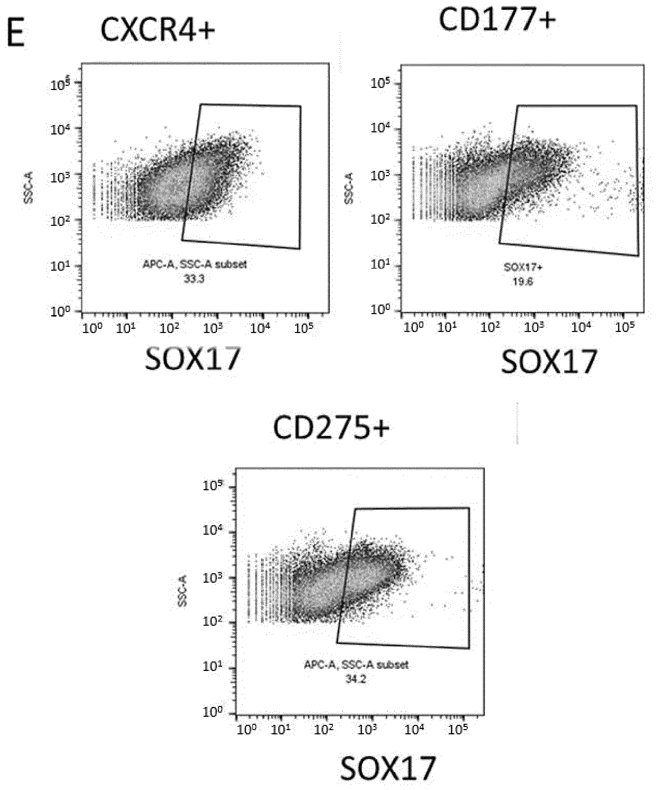

**Figure 3****: Enrichment of subpopulations can distinguish ventral and dorsal pancreatic progenitors**
   A) Schematic representation of the differentiation of hESCs towards DE and sorting using MACS and then further differentiation towards posterior gut tube
   B) Expression of ventral pancreatic progenitor marker SOX17 in CD177+, CXCR4+ and CD275+ cells. The expression of PDX1 is shown to indicate the formation of pancreatic progenitors.
   C) mRNA quantification showing higher expression of ventral pancreatic progenitor marker SOX17 in CD275+ DE compared to CD177+ DE.
   D) qPCR analysis confirming upregulation of dorsal pancreatic progenitor genes (NOGGIN, MNX1, PTCH1) in CD177+ compared to CD275+ and CXCR4+
   E) Representative FACS plots showing more expression of SOX17 in CD275+ cells compared to CD177+
**Figure 4****: CD177+ subpopulations are specified pancreatic progenitors, while CD275⁺ are liver primed progenitors**
   A) Schematic representation of the differentiation of hESCs towards DE and sorting using MACS and then further differentiation towards pancreatic progenitors
   B) qPCR quantification showing the expression of liver progenitor markers (HHEX, AFP, TTR) and pancreatic progenitor marker (PDX1)
   C) Representative images of immunofluorescence stainings of PDX1 in CD177+ and CD275+ cells
   D) Representative images of immunocytochemistry stainings showing β-CATENIN localization in CD177+ (membrane), CD275+ (cytoplasm and nuclear) and CXCR4+ (membrane).
   E-F) Representative FACS plots and graphical analysis of CD275+ and CD177+ cells stained for PI to show cell cycle status.

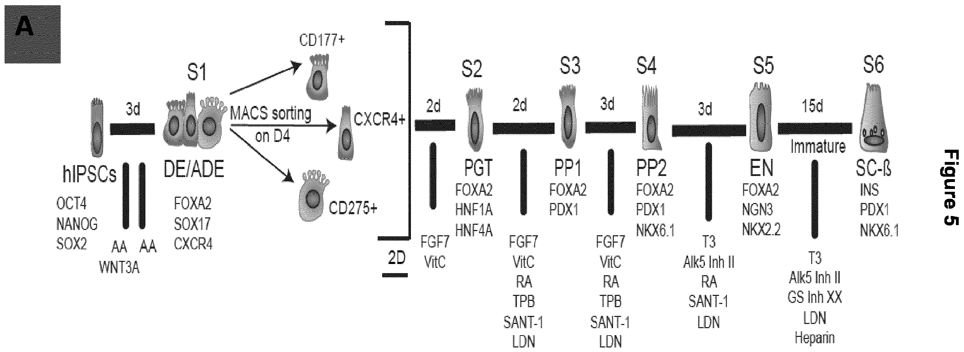

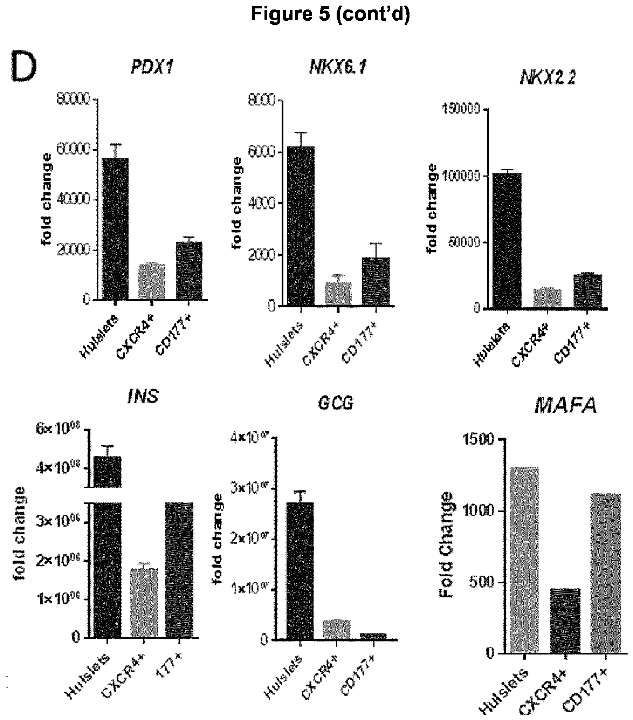

**Figure 5****: CD177+ DE generates compact, mature mono-hormonal β-cells in vitro**
   A) Schematic representation of the differentiation of hESCs towards pancreatic β-cell.
   B) Graphical representation showing the percentage of INS+ (insulin), GCG+ (glucagon) and INS/GCG double positive cells in generated by using CD177+ and CXCR4+ cells.
   C) Representative FACS plots showing percentage of cells positive for pancreatic transcription factors PDX1 and NKX6.1 and hormones INS and GLU
   D) qPCR analysis showing expression of pancreatic transcription factors like PDX1, NKX6.1, and NKX2.2 in β cells generated from CD177+ and CXCR4+ cells. mRNA quantification showing more INS and less GCG expression in CD177+ cells compared to CXCR4+ cells. Maturation marker MAFA is also upregulated in CD177+ cells compared to CXCR4+ cells.
   E) Representative images of CD177+ subpopulation showing small compact structures compared to CXCR4+ subpopulation
**Figure 6****: Identification of novel CD177+ and CD275+ ADE subpopulations**
   A) Schematic representation of hESCs differentiation towards DE showing the growth factors and small molecules added.
   B-C) Representative FACS plot of CXCR4+/CD117+ cells (b) showing a heterogeneous population and apparent homogenous FOXA2+/SOX17+ (c) DE.
   D-E) Gene expression profile of CXCR4+/CD117high, CXCR4+/CD117mid and CXCR4+/CD117low cells for *FOXA2* and *SOX17* (n=3 biological replicates for all genes, n=2 technical replicates per point).
   F-G) Gene expression profile of CXCR4+/CD117high, CXCR4+/CD117mid and CXCR4+/CD117low cells for *CER1* and *HHEX* (n=3 biological replicates for all genes, n=2 technical replicates per point). * represent the significance between the samples. Mean ± SDV, ** P < 0.05.

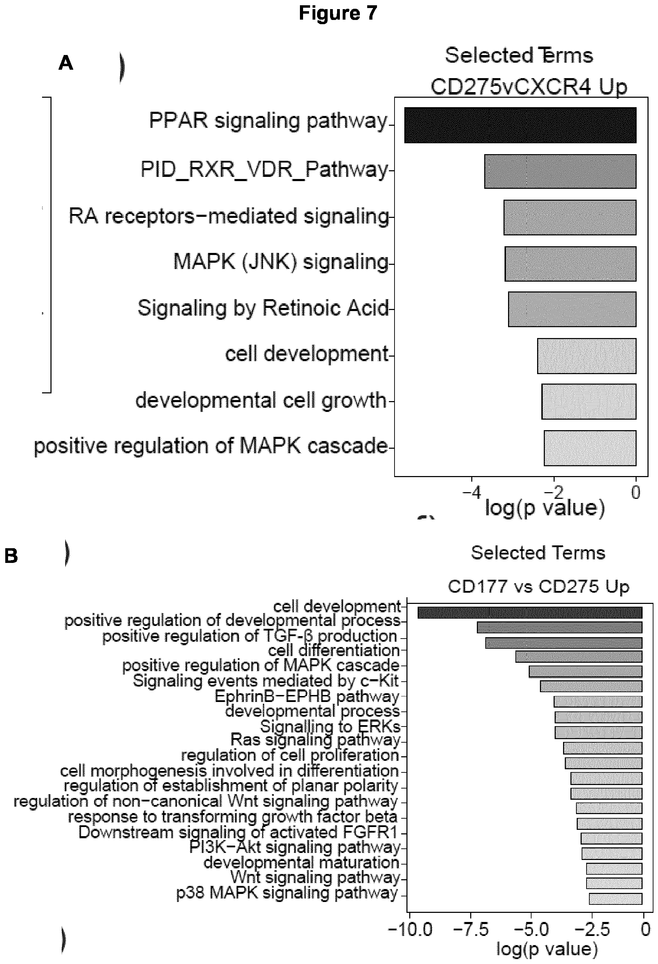

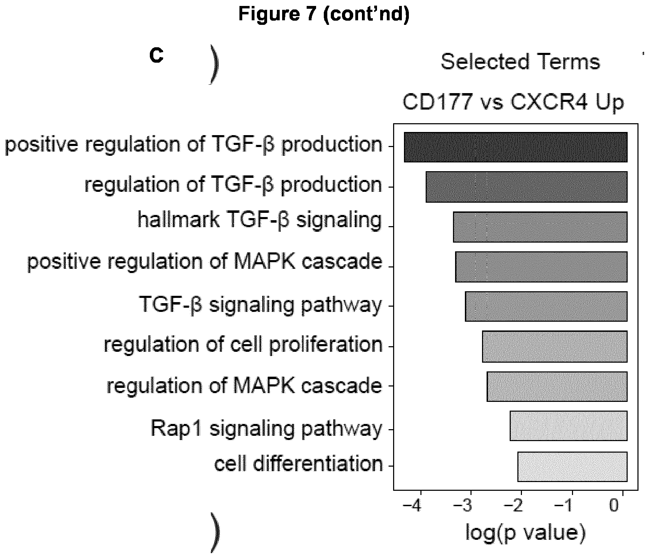

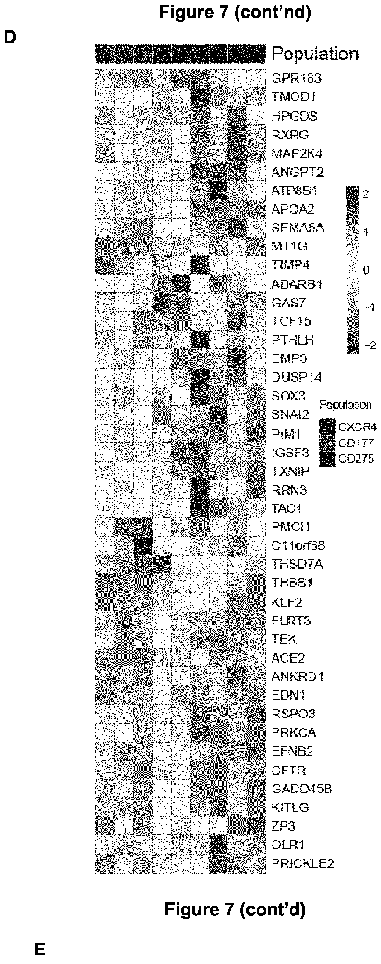

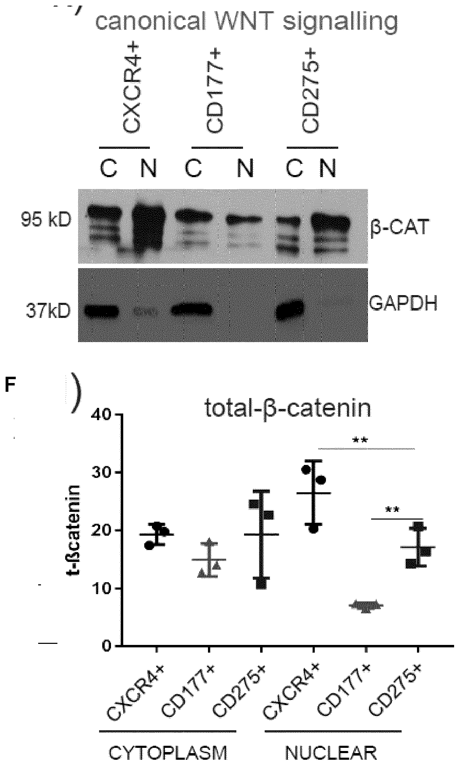

**Figure 7****: Molecular profiling of CD177+, CD275+ and CXCR4+ ADE subpopulations reveal distinct signatures**
   A-C) Bar graphs of selected and significantly enriched gene ontology (GO) terms in CD177+ versus CD275+(B), CD177+versus CXCR4+(C) and CD275+versus CXCR4+(A) DE populations.
   D) Heat map depicting differentially expressed genes in CXCR4+, CD177+and CD275+.
   E-F) Nuclear fractionation (E) and quantification (F) of β-catenin in cytoplasm (C) and nucleus (N) of enriched ADE subpopulations.

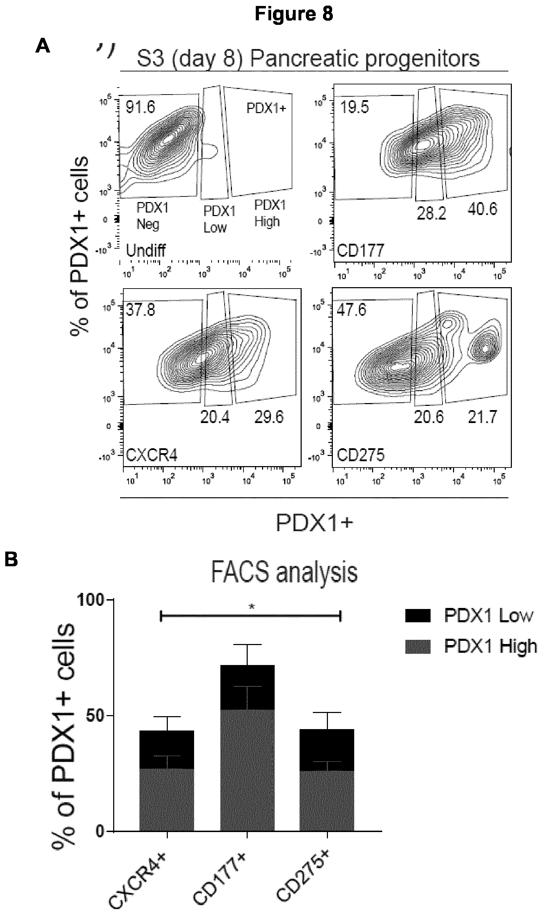

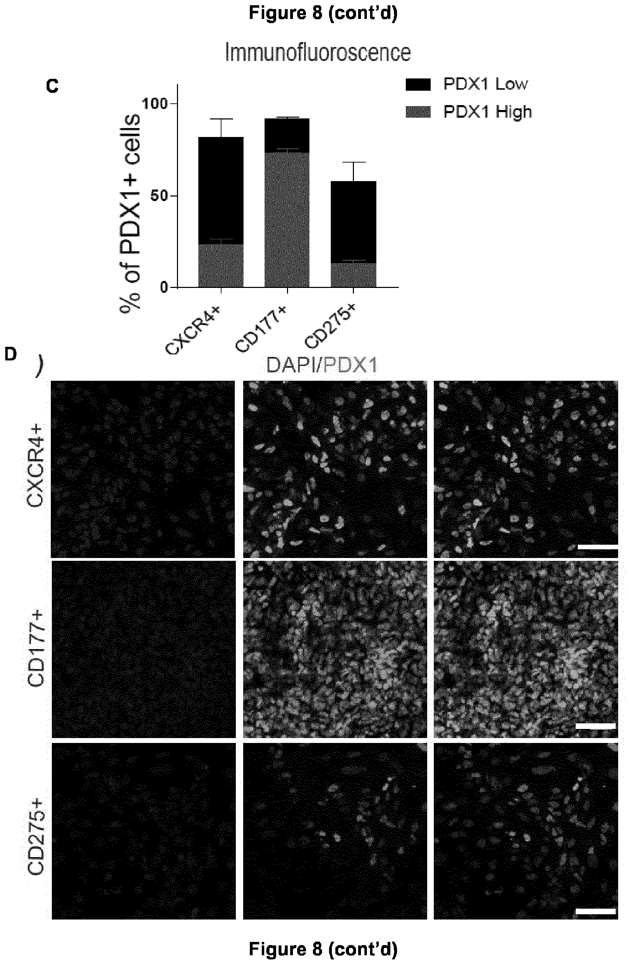

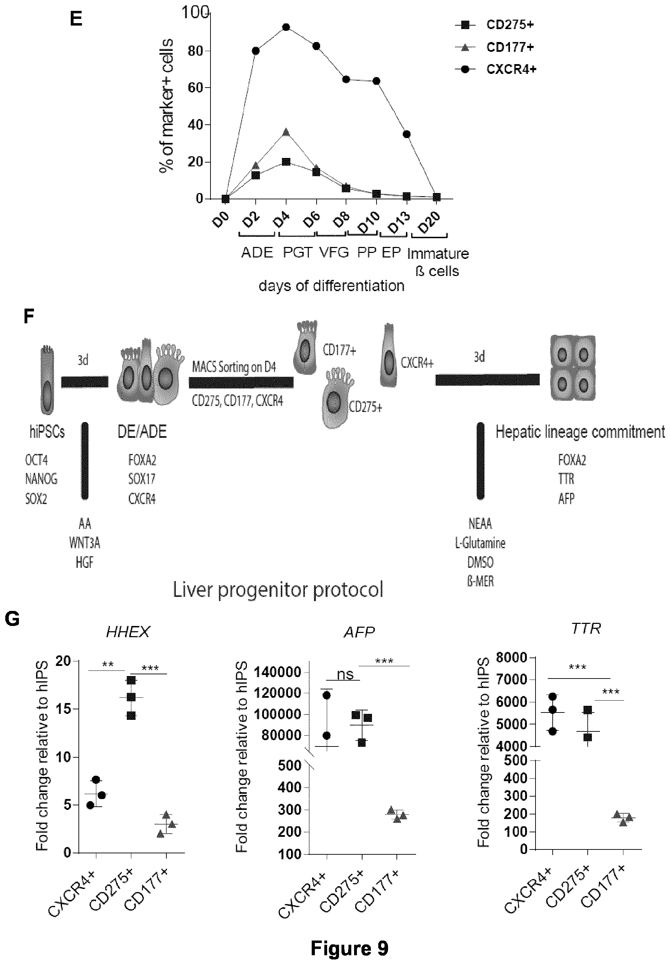

**Figure 8****. CD177+ ADE efficiently differentiates into PDX1+ pancreatic progenitors**
   A-B) PDX1 intracellular FACS analysis (A) and quantification (B) of CD177+-, CD275+- and CXCR4+-ADE derived PP showing percentage of PDX1high and PDX1low cells.
   C-D) Immunofluorescence staining (D) and analysis (C) for PDX1high and PDX1low cells in CD177+-, CD275+- and CXCR4+-ADE derived PPs. Scale bars 50 µm.
   E) Expression of CD177+-, CD275+-, and CXCR4+ during differentiation of hESCs towards pancreatic β-like cells.
   F) Liver differentiation protocol.
   G) qPCR quantification of the expression of early liver progenitor markers *HHEX, TTR* and *AFP* in enriched ADE subpopulations.
**Figure 9****: Characterisation of pancreatic progenitors derived from CD177+, CD275+ and CXCR4+ ADE cells based on GATA6 and SOX2 expression**
   A) Immunofluorescent staining of pancreatic progenitor cells derived from enriched ADE subpopulations for the co-expression of posterior foregut marker GATA6 and PDX1. Scale bars 50µm.
**Figure 10****: Characterisation of pancreatic progenitors derived from CD177+, CD275+ and CXCR4+ ADE cells based on CDX2 and PTF1A expression**
   A) Immunofluorescent staining of pancreatic progenitor cells derived from enriched ADE subpopulations for the co-expression of intestinal marker CDX2 and PDX1. Scale bars 50µm.
   B) Immunofluorescent staining of pancreatic progenitor cells derived from enriched ADE subpopulations for the co-expression of exocrine pancreatic marker PTF1A and PDX1. Scale bars 50µm.

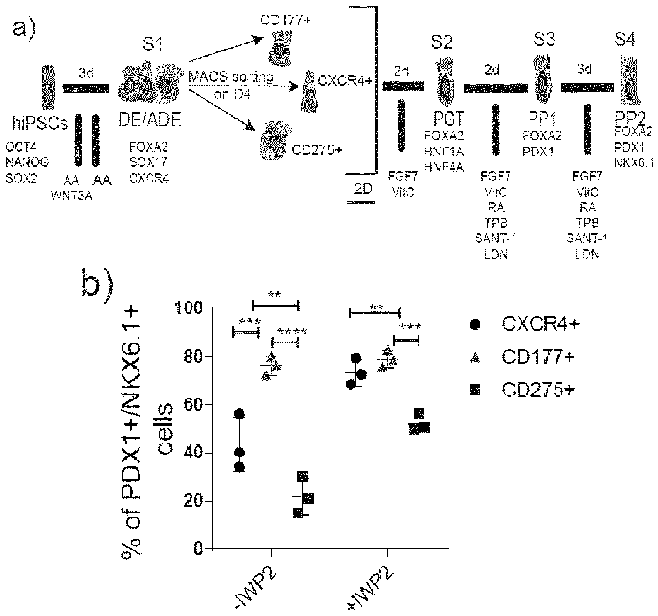

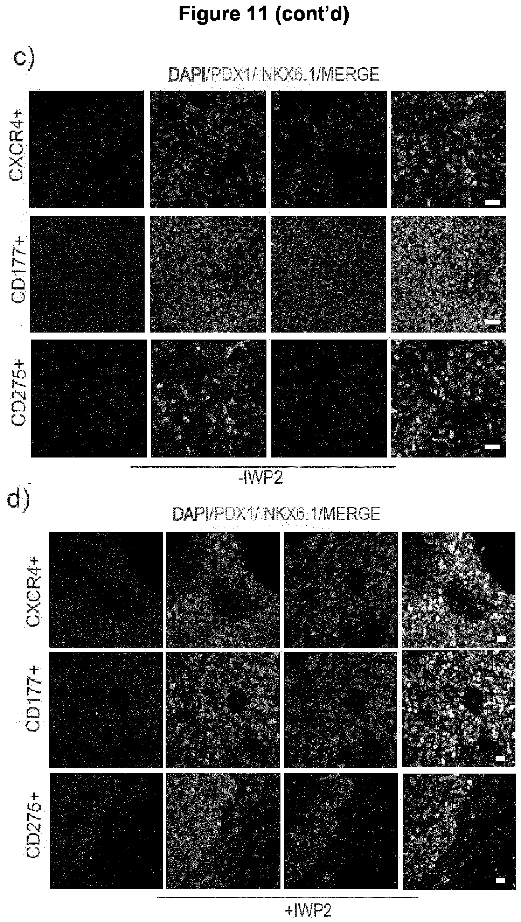

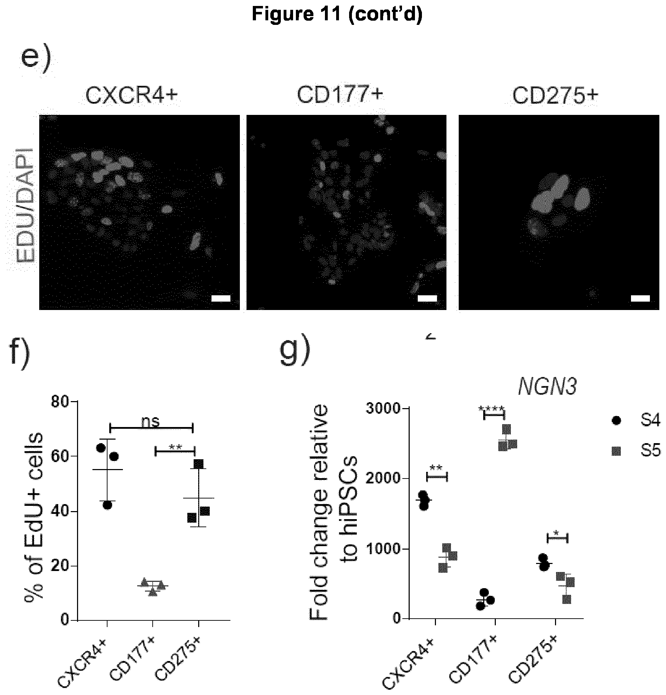

**Figure 11****: Inhibition of WNT secretion promotes pancreatic differentiation**
   A) Overview of differentiation protocol towards pancreatic progenitors where inhibition of WNT secretion is performed at S2 stage using IWP2
   B-D) FACS quantification (B) and immunocytochemistry (C-D) for the percentage of cells expressing PDX1+/NKX6.1+ generated from CD177+-, CD275+- and CXCR4+-ADE before (B, C), Scale bars, 50 µm and after (B, D) the treatment with IWP2. Scale bars, 20 µm.
   E-F) EdU staining (E) and quantification (F) for EdU+ cells in different ADE subsets revealing the exit of cells from the cell cycle. Scale bars, 80 µm. (G) mRNA analysis of *NGN3* at S4 and S5 stage in different ADE subpopulations. (n=3, biological replicates). Error bar represents +/- S.D; *P < 0.05, **P < 0.01, two-sided unpaired t-test. Data were normalized to 18S.

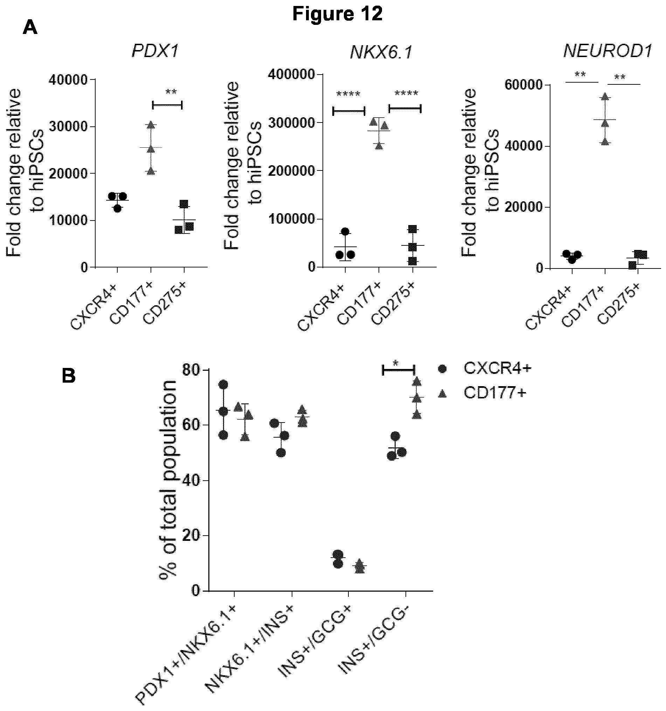

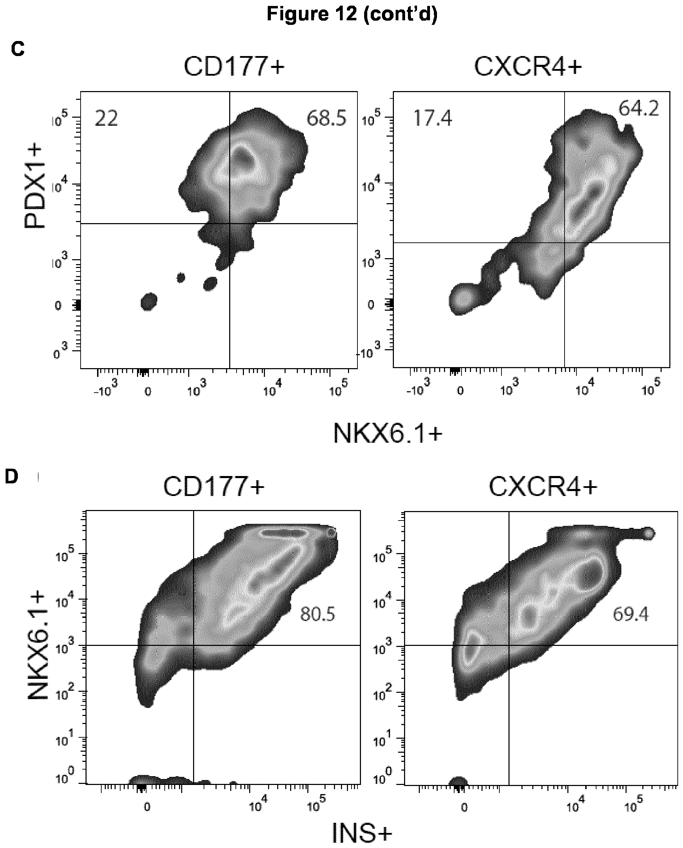

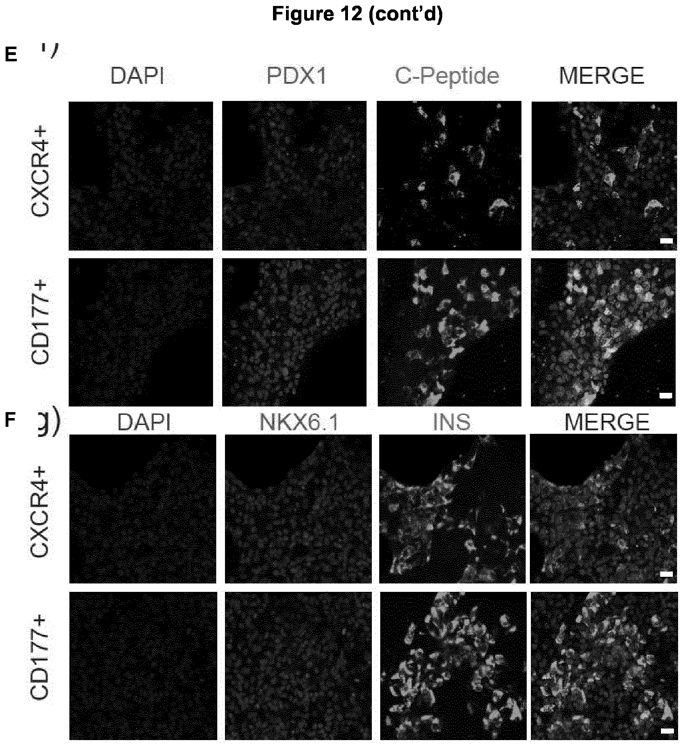

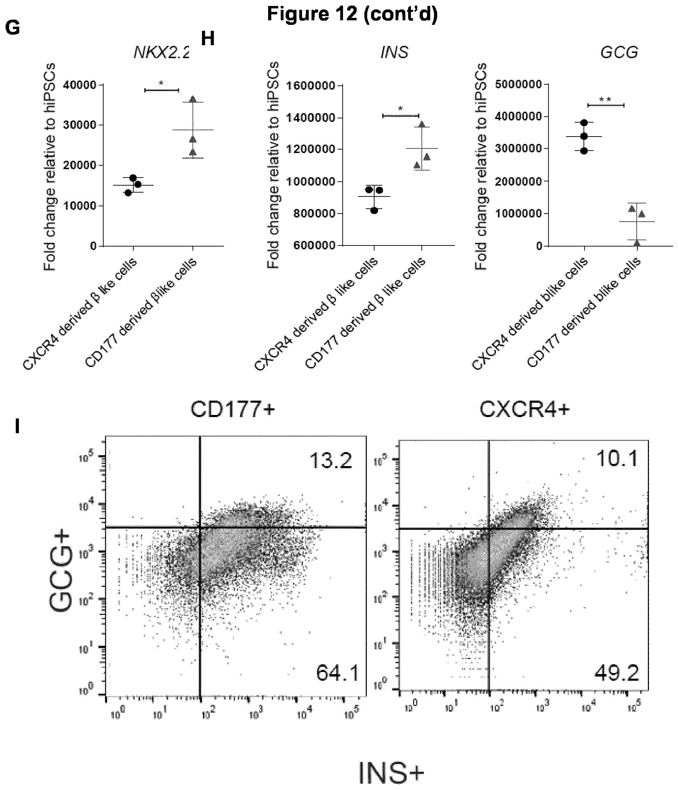

**Figure 12****: CD177+- enriched DE cells efficiently generate NKX6.1+/INS+ β-like cells**
   A) qPCR gene expression of S6 cells generated from CD177+- and CXCR4+-ADE for the expression of *PDX1, NKX6.1* and *NEUROD1.* Error bar represents +/- S.D; *P < 0.05, **P < 0.01, two-sided unpaired t-test. Data were normalized to 18S.
   B-C) Representative FACS plots for CD177+- and CXCR4+- ADE-derived β-cells for expression of PDX1 and NKX6.1 (B) and NKX6.1 and INS (C).
   D) FACS quantification of the cells at S6 stage for the percentage of cells expressing various transcription factors and pancreatic hormones.
   E-F) Representative immunofluorescent staining for S6 cells derived from enriched CD177+ and CXCR4+ DE for: (E) PDX1 and C-peptide and (F) NKX6.1 and INS. Nuclear DAPI staining is shown as well. Scale bars, 50 µm.
   G) mRNA quantification of sorted subpopulations derived β-like cells for pancreatic endocrine transcription factors such as *PDX1, NKX6.1* and *NKX2.2* in comparison to human islets.
   H) Quantification of mRNA transcripts of pancreatic hormones INS and GCG in β- like cells generated from enriched subpopulations.
   I) Representative FACS plots of stage 6 cells generated from enriched ADE subpopulations for the expression of INS and GCG to mark poly-hormonal cells.

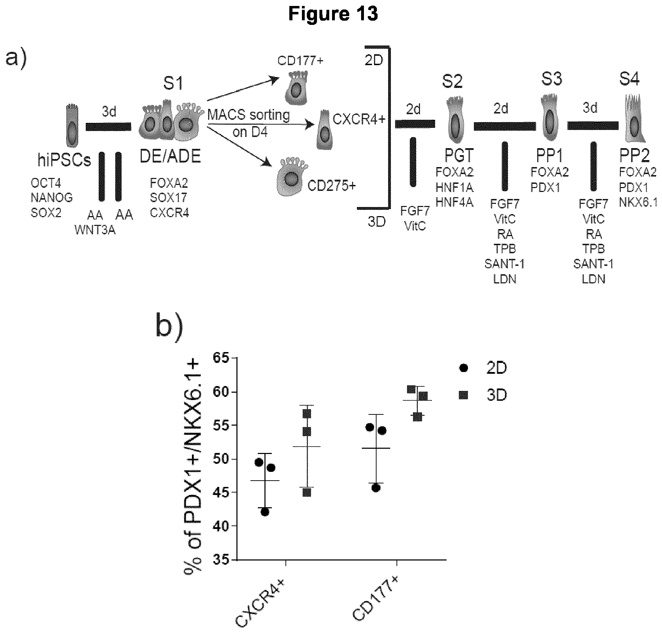

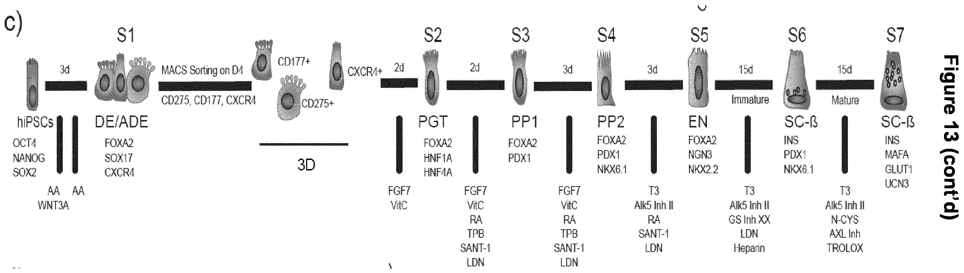

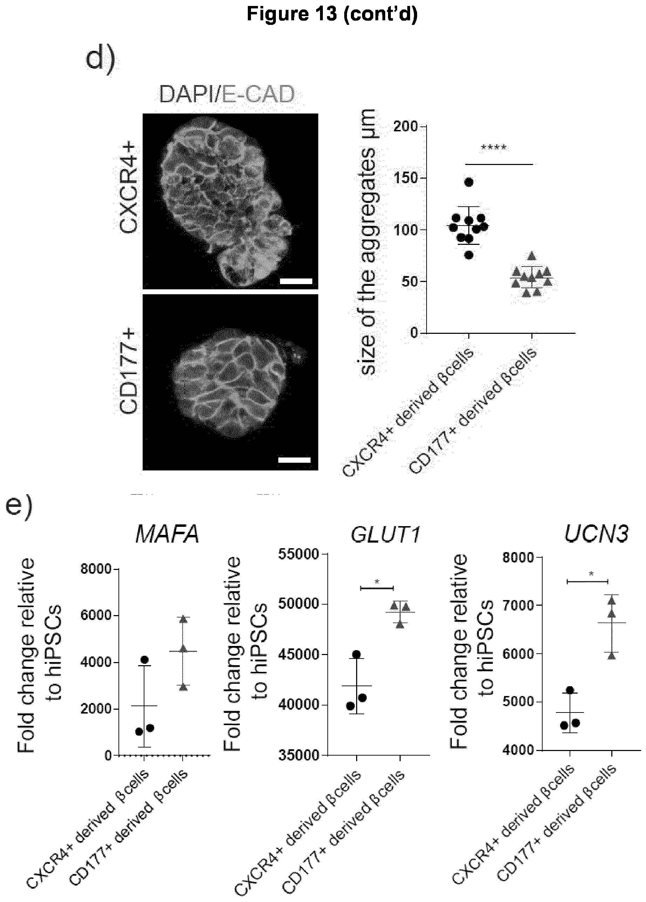

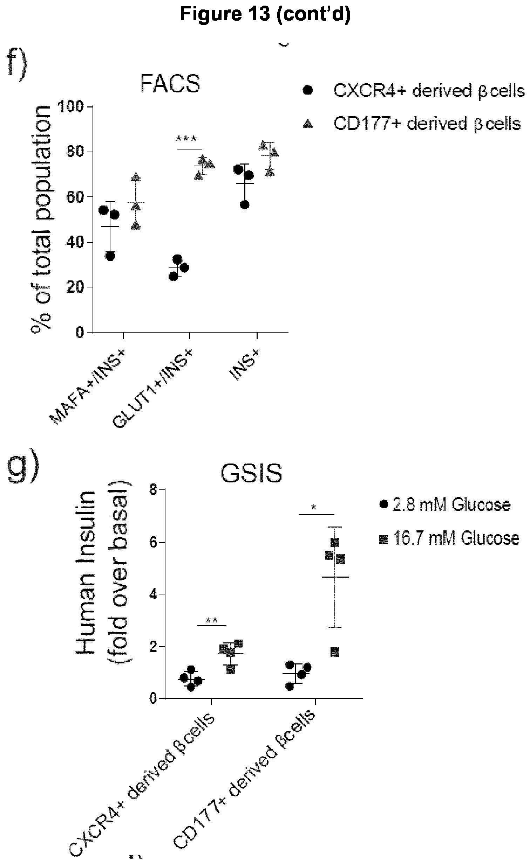

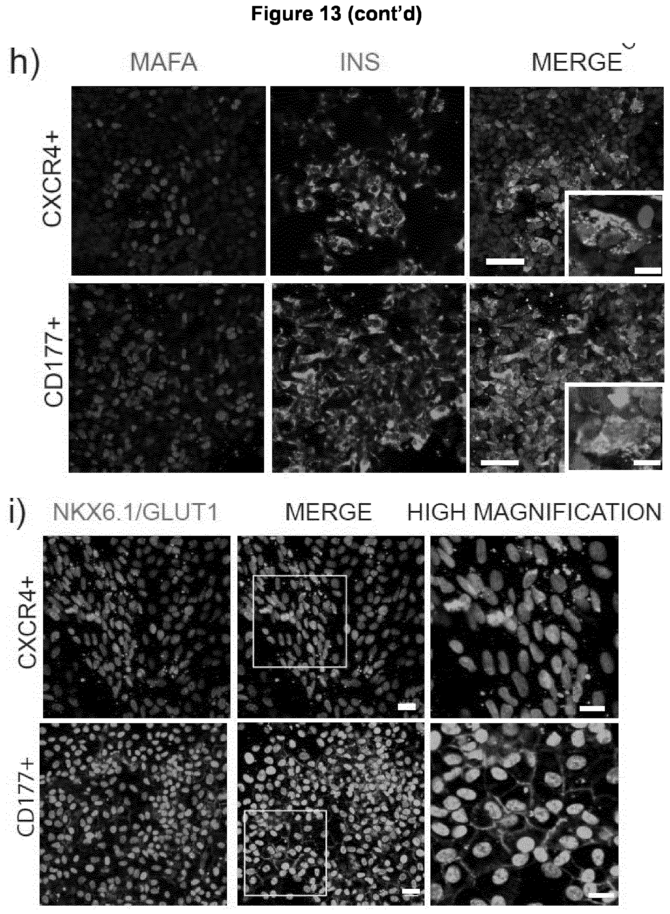

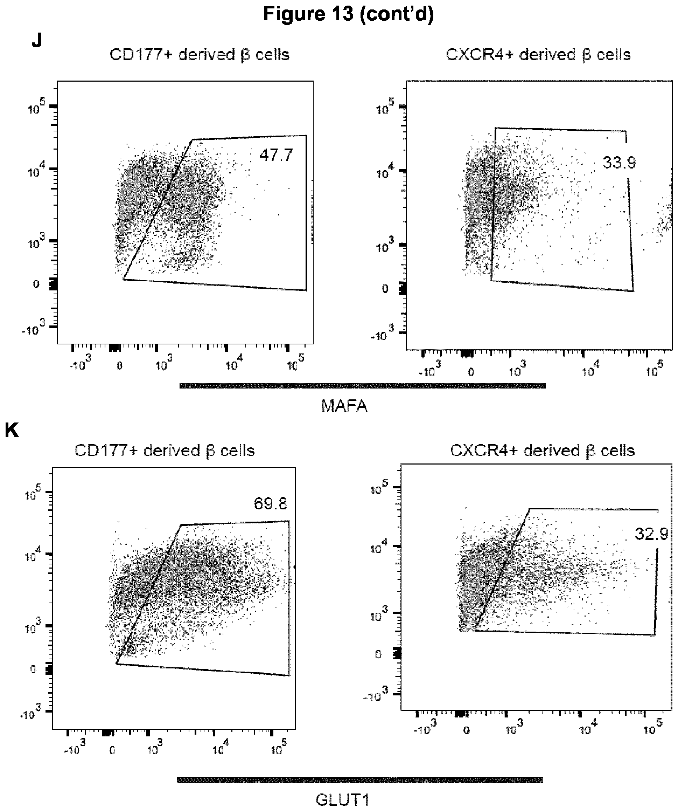

**Figure 13****: CD177+- enriched ADE generate more β-like cells *in vitro.***
   A) Outline of the differentiation protocol comparing 2D vs 3D.
   B) Comparison of PDX1+/NKX6.1+ generated from CD177+-, CD275+- and CXCR4+-ADE in 2D and 3D settings
   C) Illustration of differentiation protocol to generate β-like cells in clusters from CD177+- and CXCR4+-ADE.
   D) Morphology of CD177 and CXCR4-derived β-like cells; DAPI (blue) and E-CAD (green). Scale bars, 20 µm. Graph represents the size of the aggregates in µm.
   E) Gene expression profile of β-like cells for maturation markers *MAFA, GLUT1* and *UCN3.* Data are expressed as the fold change relative to hiPSCs. Error bar represents +/- S.D; **P* < 0.05, ***P* < 0.01, two-sided unpaired t-test. Data were normalized to 18S.
   F,H-I) FACS quantification (F) and immunohistochemistry for the expression of MAFA (left), INS (middle). Insets show higher magnification images. (H) and NKX6.1 (green, nuclear) and GLUT1 (green, membrane) (I).DAPI is shown in blue. Scale bars, in low magnification images, 50 µm and high magnification images 5 µm.
   G) Static glucose stimulated human insulin secretion by β-like cells derived from CD177+ and CXCR4+ ADE (n=4, including five technical replicates per batch). Data expressed as the fold change relative to basal insulin levels and normalized to number of cells.
   J-K) Representative FACS plots for β cell maturation markers MAFA (J) and GLUT1 (K) in S7 cells generated from CD177+ and CXCR4+ ADE cells.

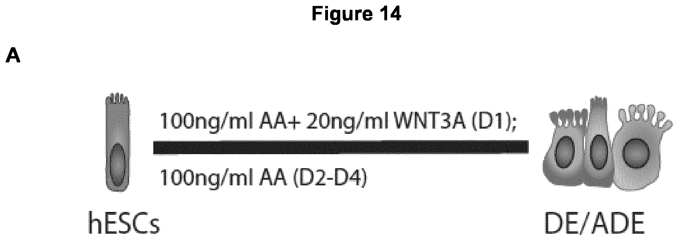

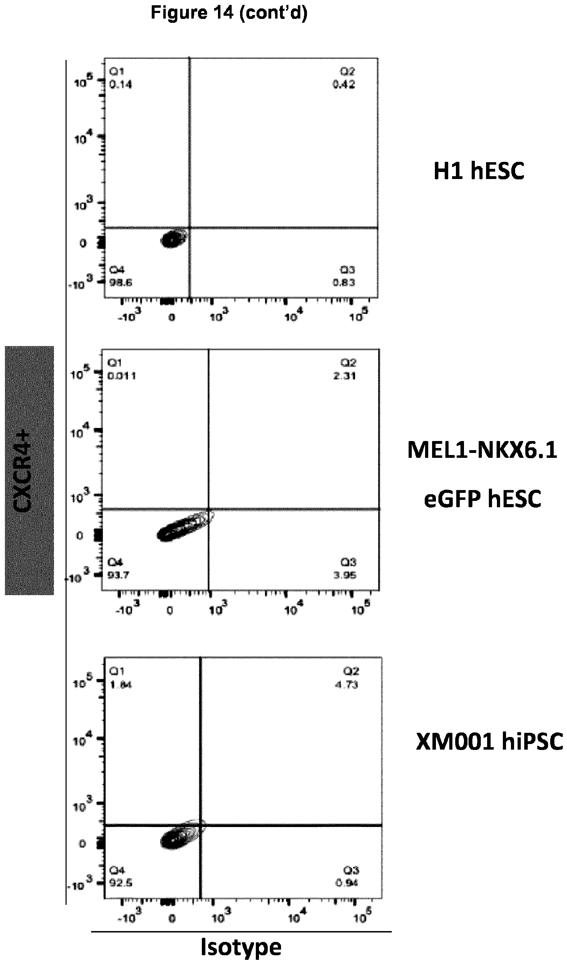

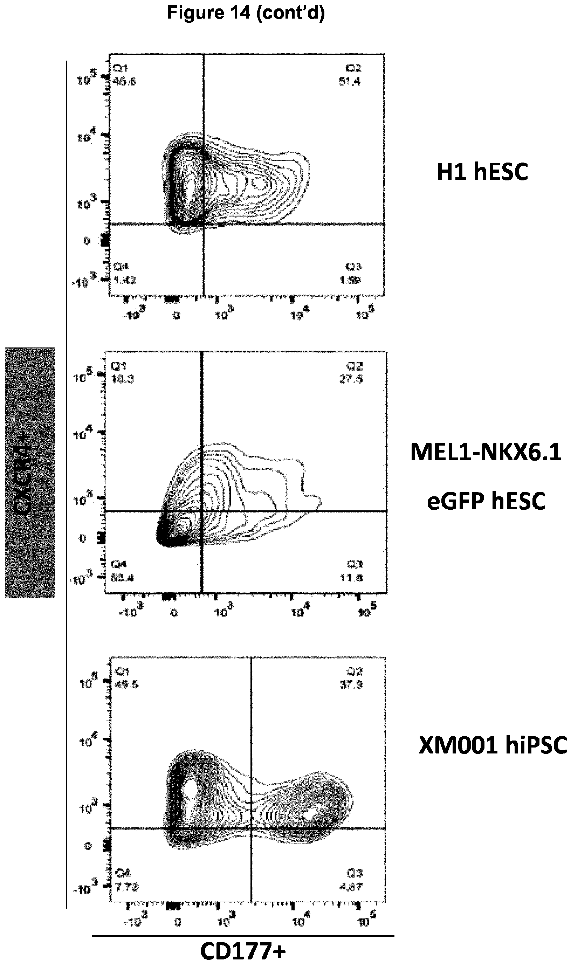

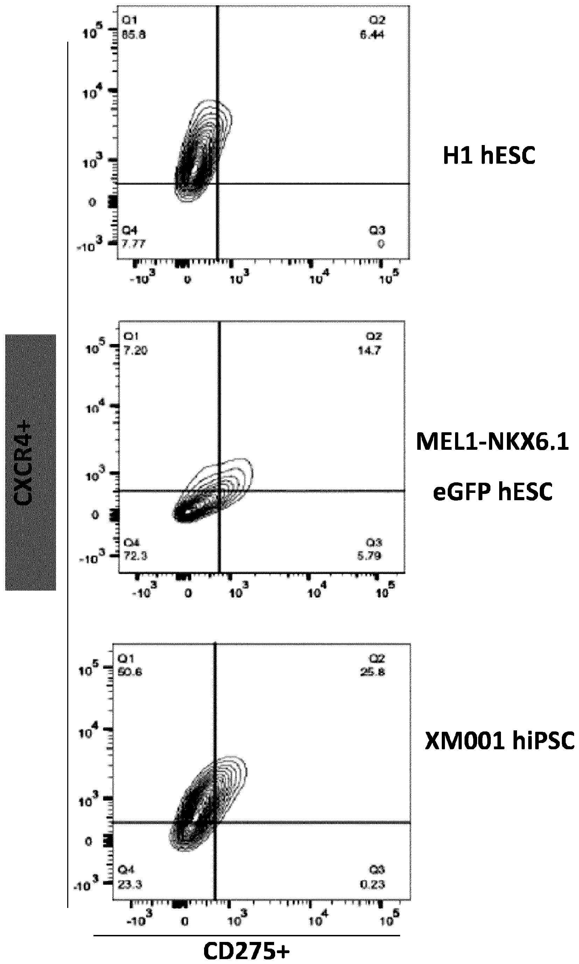

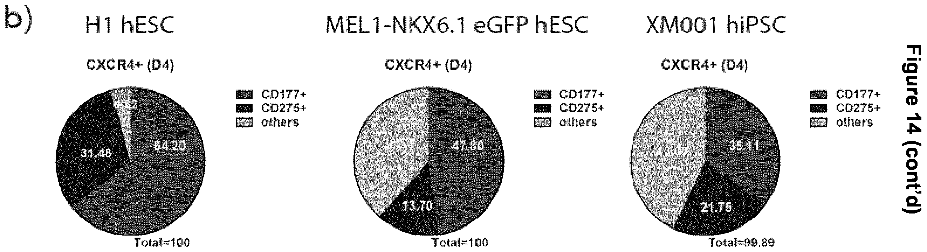

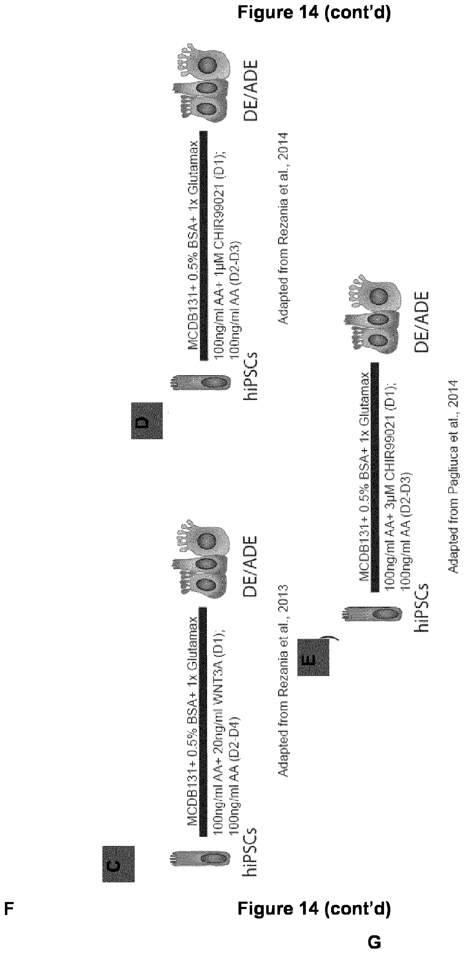

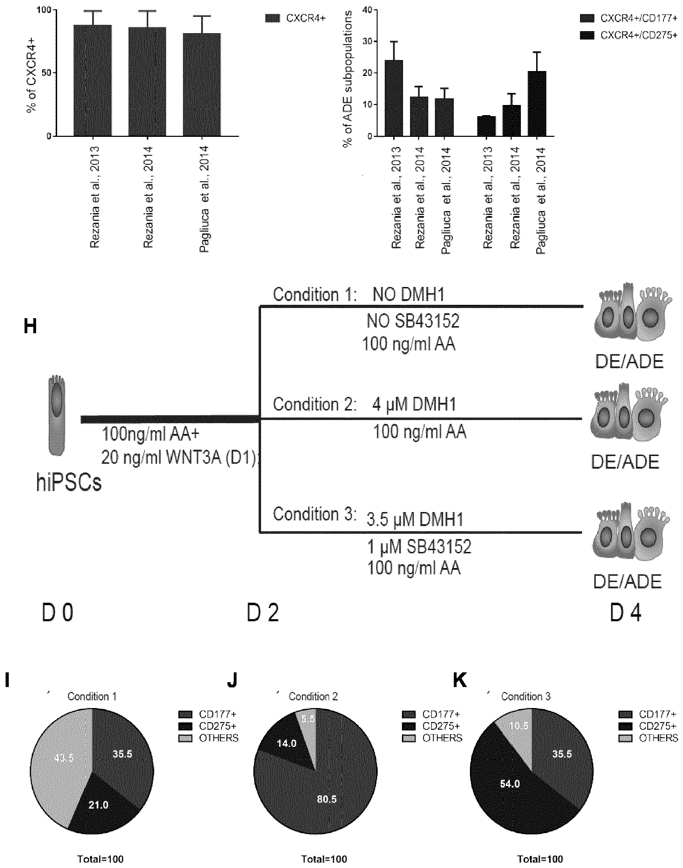

**Figure 14****: Induction efficiency by changing parameters**
   A-B) Endoderm differentiation scheme from hESCs towards DE/ADE. FACS plots (A) represent the percentage (B) of CXCR4+/CD177+ and CXCR4+/CD275+ subpopulations in hH1, hMEL1-NKX6.1 and hiPSC at DE/ADE stage.
   C-E) Adaptation of previously published endoderm differentiation protocols from hESCs.
   F) FACS quantification for the percentage of total population expressing CXCR4 in DE cells derived from iPSCs cells using 3 different endoderm induction protocols (n=3 biological replicate).
   G) FACS quantification for the percentage of cells expressing CXCR4+/CD177+ and CXCR4+/CD275+ in DE generated using previously published protocols.
   H) Schematic representation dose dependent inhibition of TGF-β and BMP signalling during induction of DE.
   I-K) Pie charts representing the percentage of CD177+ and CD275+ ADE subpopulations generated using no inhibition of pathways (I, condition 1), with high inhibition of BMP signalling using BMP inhibitor DMH1 (J, condition 2) and with moderate BMP inhibition and low TGF-β inhibition (K, condition 3).

### DETAILED DESCRIPTION

The following description includes information that may be useful in understanding the present invention. It is not an admission that any of the information provided herein is prior art or relevant to the presently claimed inventions, or that any publication specifically or implicitly referenced is prior art.

### Definitions

It will be appreciated that the numerical ranges expressed herein include the endpoints set forth and describe all integers between the endpoints of the stated numerical range. Singular claim terms are plural in scope unless there is clear intent to the contrary.

"Pluripotent stem cells" as used herein are cells that can give rise to each of the three embryonic cell lineages forming the three germ layers as well as extraembryonic cells. Those germ layers are endoderm, mesoderm and ectoderm. In the context of this invention, cells of the endodermal lineage forming pancreatic progenitor cells and/or liver progenitor cells are preferred. Pluripotent cells, however, may not be capable of producing an entire organism.

In certain embodiments, the pluripotent cells used as starting material are stem cells, including human embryonic stem cells As used herein, "embryonic" refers to a range of developmental stages of an organism beginning with a single zygote and ending with a multicellular structure that no longer comprises pluripotent or totipotent cells other than developed gametic cells. Human embryonic stem cells can be isolated from embryos without destruction as outlined in WO 03/046141 and Young 2008.

As used herein, "induced pluripotent stem cells" (iPSC) are cells that can be directly converted from differentiated or adult cells to a type of pluripotent stem cells. The production of iPSC was pioneered by Takahashi et al. 2006 and gives rise to a cell type very similar to ESC or PSC.

Fig. 5A shows an overview of the differentiation from stem cells to beta cells. The different stages and responding cell populations will be defined in the following:
"Pancreatic progenitor cells" (PP) as used herein are generally cells capable of differentiating into endocrine precursor cells and further to pancreatic beta cells, see e.g. Fig. 5A. Accordingly, PP includes ADE, PGT, PP1, PP2, EN and SC-β. As used herein, "definitive endoderm" (DE) refers to a multipotent cell that can differentiate into cells of the gut tube or organs derived from the gut tube, like e.g. pancreas or liver. In accordance with certain embodiments, the definitive endoderm cells and cells derived therefrom are mammalian cells, and in a preferred embodiment, the definitive endoderm and/or anterior definitive cells are human cells. The phrase "anterior definitive endoderm" (ADE) as used herein describes a subpopulation of the DE especially capable of the development into cells of the pancreatic lineage (see Example 2) and is characterized by an increased Wnt and non-canonical planar cell polarity (PCP) signaling as outlined in Example 2. Markers defining the ADE comprise FOXA2, SOX17 and CER1. The cells of the ADE are preferably used in the method of purifying a pancreatic progenitor cell according to the invention. Preferably, the pancreatic progenitor cell is a cell of the ADE, the DE, the PGT and/or the VFG. More preferably, the pancreatic progenitor cell is a cell of the ADE. Preferably, the pancreatic progenitor cell preferably comprises CD177 on the cell surface.

"Posterior gut tube" (PGT) as used herein refers to an intermediate cell population. It is characterized by the expression of the markers FOXA2, HNF1alpha (HNF1A), HNF4alpha (HNF4A).

Two further cell populations as used herein are "pancreatic progenitor cells stage 1" (PP1) and "pancreatic progenitor cells stage 2" (PP2). They represent an intermediate cell population in the development of the pancreatic lineage. Typical markers for PP1 comprise FOXA2 and PDX1 and typical markers for PP2 comprise FOXA2, PDX1 and NKX6.1.

Pancreatic progenitor cells (PP) can further differentiate to a pancreatic-lineage cell expressing neurogenin 3 (NGN3) herein referred to as "endocrine cells" (EN). Typical markers for this cell type comprise FOXA2, NGN3 and NKX2.2.

As used herein, the term "pancreatic beta cells" (SC-β) refers to endocrine cells, which are capable of secreting a hormone and are also responsive to various agents that stimulates or effects the secretion of such hormone, in vitro or in vivo. For example, a "mature" endocrine cell as described herein is a cell which is, for example, glucose responsive and releases insulin in a physiologically appropriate manner. As used herein, the term "immature" refers to a cell, which is not fully capable of secreting hormones, or, in other embodiments, not fully capable of secreting hormones due to various stimuli in vitro.

With respect to additional aspects of the embodiments described herein, the PSC/iPSC/ESC-derived cell types described herein can be enriched, depleted, isolated, separated, sorted and/or purified as further described in the examples. As used herein, the terms "enriched" or "purified", or enriched or purified due to depletion of other known cell populations, indicate that the cell population has been subject to some selection process so that the population is enriched and/or purified. Also, the subject cells are also considered relatively enriched and/or purified, i.e. there is significantly more of a particular PSC/iPSC/ESC-derived cell type population as compared to another PSC/iPSC/ESC-derived type population or as compared to the original or initial cell culture. That said, sometimes enriching or purifying for particular PSC/iPSC/ESC-derived cells types can involve "depleting" or "separating" or "sorting" one or more known PSC/iPSC/ESC-derived cell type from another PSC/iPSC/ESC-derived cell type. For example, in some embodiments, certain PSC/iPSC/ESC-derived cell types are enriched or purified because they do not bind or cross-react to a ligand and/or agent and/or antibody and/or antibody bound to solid or semi-solid matrix. Such enriched or purified populations can be said to be "depleted" or "separated" or "sorted" from the PSC/iPSC/ESC cell culture. As a further example, such depleted or separated or sorted PSC/iPSC/ESC populations can be found or isolated in the flow-through fractions in a standard affinity chromatography method; or stated in another way, the non-binding fractions. Accordingly, in certain embodiments, it is advantageous to enrich and purify a PSC/iPSC/ESC-derived cell type by depleting the culture of known or unknown cell types. In this way, the enriched or purified cell population does not have the bound or attached antibody. Because there is no need to remove the antibody from the purified population, the use of the enriched or purified cells for cell therapies is improved.

In certain embodiments, the terms "enriched", "isolated", "separated", "sorted", "purified" or equivalents thereof refer to a cell culture or a cell population or cell sample that contains at least about 30%, 40%, 50%, 55%, 60%, 65%, 70%, 75%, 80%, 85%, 90%, 95%, or 100% of the desired cell lineage or a desired cell having a certain cell phenotype, e.g., expressing a certain cell marker or not expressing a certain cell marker gene characteristic of that cell phenotype.

As used herein, "ligand" refers to a moiety or binding partner that specifically binds or cross-reacts to the marker or target or receptor or membrane protein on the cell or to the soluble analyte in a sample or solution.

As used herein, "marker", "epitope", "target", "receptor" or equivalents thereof can refer to any molecule that can be observed or detected. For example, a marker comprises a nucleic acid, such as a transcript of a specific gene, a polypeptide product of a gene, such as a membrane protein, or a non-gene product. A "cell surface marker" is a marker present at the cell surface.

As used herein, a "capture medium" refers to any matrix or medium which allows it to be separated from the cell population sample, for example, a physiologically compatible bead. Characteristics of such a medium include refractive index, size, light scatter intensity, magnetism or carrying a fluorescent detector dye to provide a unique fluorescent signature. For example, one subset of solid phase capture medium includes stable colloidal particles, such as beads ranging in size from between about 0.05 to about 10 µm in diameter (i.e., colloidal-sized). Such beads can contain dextran, aminodextran, aldehyde and/or sulfate functional groups. Preferably, a capture medium comprises a bead.

CD51, also known as integrin alpha V and vitronectin receptor alpha subunit, is a member of the integrins, a family of heterodimeric glycoproteins involved in cell-cell adhesion and in binding to basement membrane and extracellular matrix ligands. CD51 is part of the receptor for fibronectin, vitronectin, fibrinogen and other proteins. CD51's UniProt Accession number is P06756 and its HUGO number is HGNC:6150.

CD177, also known as Human neutrophil alloantigen 2a (HNA-2a), NB1 glycoprotein (NB1GP) or Polycythemia rubra vera protein 1 (PRV-1), is a glycosyl-phosphatidylinositol (GPI)-linked cell surface glycoprotein that plays a role in neutrophil activation. The protein can bind platelet endothelial cell adhesion molecule-1 and function in neutrophil transmigration. Its UniProt Accession number is Q8N6Q3 and its HUGO number is HGNC:30072.

CD275, also known as Inducible T-Cell costimulator Ligand (ICOS ligand), B7 homolog 2 (B7-H2), B7-like protein GI50 or B7-related protein 1 (B7RP-1), is a ligand for the T-cell-specific cell surface receptor ICOS. Acts as a costimulatory signal for T-cell proliferation and cytokine secretion; induces also B-cell proliferation and differentiation into plasma cells. Its UniProt Accession number is 075144 and its HUGO number is HGNC:17087.

### Detailed description of the embodiments

Described herein are methods for identifying and characterizing various pluripotent stem cell (PSC)-, induced pluripotent stem cell (iPSC)- and/or embryonic stem cell (ESC)-derived cell types using ligands which cross-react with cell surface markers. Cell surface markers may be receptors, membrane proteins and/or epitopes. Preferred cell surface markers are CD51, CD177 and CD275. Preferably, the identified and/or characterized cell types are pancreatic progenitor cells, identified using the cell surface marker CD177. Further described herein are compositions of pancreatic progenitor cells resulting from the methods in accordance with the present invention. ESCs are in a preferred embodiment human embryonic stem cells (hESC). General methods for production of PSC/iPSC/ESC-derived cells are described in D'Amour 2005, D'Amour 2006, Rezania 2014, Pagliuca 2014 and Kroon 2008. The preferred protocol of this invention is the protocol of Rezania 2014. This protocol is also shortly summarized in Fig. 5A.

Accordingly, the present invention relates to a method of purifying a pancreatic progenitor cell comprising:
(a) exposing a population of cells derived from pluripotent stem cells, induced pluripotent stem cells and/or embryonic stem cells comprising a pancreatic progenitor cell, to a ligand which binds to a cell surface marker on the pancreatic progenitor cell, wherein the cell surface marker is CD177 and
(b) separating the pancreatic progenitor cell from cells derived from pluripotent stem cells, induced pluripotent stem cells and/or embryonic stem cells which do not bind to the ligand, thereby purifying said pancreatic progenitor cell,
wherein the pancreatic progenitor cell is sorted at the stage of the definitive endoderm (DE), the anterior definitive endoderm (ADE), the posterior gut tube (PGT) and/or the ventral foregut (VFG).

The method of the present invention may also be understood as a method of purifying a subpopulation of pancreatic progenitor cells, such as a specified (A)DE subpopulation towards pancreatic lineage. This method of purifying a subpopulation of pancreatic progenitor cells, such as a specified (A)DE subpopulation towards pancreatic lineage, cells may comprise: (a) exposing a population of cells derived from pluripotent stem cells, induced pluripotent stem cells and/or embryonic stem cells comprising a specified (A)DE cell, to a ligand which binds to a cell surface marker on the specified (A)DE cell, wherein the cell surface marker is CD177 and (b) separating the specified (A)DE cell from cells derived from pluripotent stem cells, induced pluripotent stem cells and/or embryonic stem cells which do not bind to the ligand, thereby purifying specified (A)DE cell. Specified subpopulations towards pancreatic lineage are subpopulations that preferably develop into cells of the pancreatic lineage.

For the convenience of the reader, an exemplified non-limiting procedure of isolating a subpopulation of definitive endoderm (DE) using cell surface marker CD177 for an efficient generation of pancreatic cells will be described. In Fig. 5A is depicted an overview of the procedure: First, PSCs are differentiated towards DE using the protocol of Rezania 2014. Second, the resulting DE is then sorted for CD177-positive cells - for example by MACS. These CD177-positive cells, which can also be described as a subpopulation of the ADE cells, are then thirdly used for a further differentiation towards pancreatic beta-cells.

For the purification of pancreatic progenitor cells in the method of the invention, the pancreatic progenitor cell, which may be specified ADE cells towards pancreatic lineage, should express CD177 on the surface. Pancreatic progenitor cells, which do not express CD177 on the surface, are not encompassed by the invention. As shown in Fig. 8E, pancreatic progenitor cells at the stage of DE/ADE, PGT or the VFG express CD177. Thus, the purification of pancreatic progenitor cells is carried out at the stage of DE/ADE, PGT or the VFG, more preferably at the stage of DE/ADE or most preferably on the stage of ADE. This allows the sorting of relevant subpopulations that tend to differentiate into cells of the pancreatic lineage very early, i.e. before the fate of the cells can be determined by markers known in prior art.

Accordingly, in one preferred embodiment, the pancreatic progenitor cell is a cell of the ADE. In another embodiment, the pancreatic progenitor cell is a cell of the ADE and/or the PGT. In another preferred embodiment, the pancreatic progenitor cell is a cell of the DE, the ADE, the PGT and/or the VFG (ventral foregut).

The invention is especially useful when isolating pancreatic progenitor cells at the stage of DE or the ADE. Here, the differences in CD177 expression between the (A)DE subpopulation tending to differentiate into the pancreatic lineage, and other subpopulations tending to differentiate into other cell types, for example into the liver lineage, are the highest (see also Fig. 8E). However, also the cells of the PGT or the VFG express CD177 and CD275. Accordingly, the cells derived from pluripotent stem cells, induced pluripotent stem cells and/or embryonic stem cells are cells of the ADE, the DE, the PGT and/or the VFG. In one preferred embodiment, the cells derived from pluripotent stem cells, induced pluripotent stem cells and/or embryonic stem cells are cells of the ADE.

Purifying pancreatic progenitor cells, or more precisely, a subpopulation of the ADE that is specified for the pancreatic lineage, can be seen as an aim of the present invention. Therefore, the pancreatic progenitor cells purified in accordance with the method of the present invention preferably expresses FOXA2, SOX17, CXCR4 and/or CER1. More preferably, the pancreatic progenitor cells purified in accordance with the method of the present invention expresses CER1. Alternatively, the pancreatic progenitor cell purified in accordance with the method of the present invention may express FOXA2, SOX17 and/or CXCR4. An increased expression of PDX1 of CD177⁺ cells compared to CXCR4⁺ (CD184) cells, a marker known in prior art, was observed after further differentiation of the subpopulation of the ADE (Example 5, Fig. 5C). The pancreatic progenitor cells purified in accordance with the method of the present invention may express pancreatic and duodenal homeobox gene 1 (PDX1).

In one embodiment, the population of cells derived from PSC/iPSC/ESC is additionally contacted with ligands which bind CD51 and/or CD275. Depending on the way of separating the cells, the order of ligands used for the differentiation varies. In a preferred environment, cells expressing CD275 that are prone to become cells of the liver lineage (see Examples 4 and 7) can be depleted from a population of cells derived from PSC/iPSC/ESC. Depletion in this context means the removal of those cells from a population that is only capable of differentiating in an undesired cell type. Thereby, cells of the pancreatic lineage are enriched. In another preferred environment, the cell surface marker CD51 is used in addition to CD177 to enrich cells of the pancreatic lineage. In flow cytometry, all three markers can be applied simultaneously wherein in other methods of purification like magnetic bead sorting (magnetic cell separation (MACS)) depletion and enrichment have to be carried out in two or more separate steps.

In a preferred embodiment of this invention, the ligand is an antibody or an antibody fragment. Antibodies or antibody fragments comprise immunoglobulin molecules and immunologically active portions of immunoglobulin (Ig) molecules, i.e., molecules that contain an antigen binding site that specifically binds (immuno-reacts with) an antigen. Such antibodies or fragments include polyclonal antibodies from any native source, and native or recombinant monoclonal antibodies of classes IgG, IgM, IgA, IgD, and IgE, hybrid derivatives, and fragments of antibodies including Fab, Fab' and F(ab')2, humanized or human antibodies, recombinant or synthetic constructs containing the complementarity determining regions of an antibody, an Fc antibody fragment thereof, a single chain Fv antibody fragment (scFv), a synthetic antibody or chimeric antibody construct which shares sufficient complementarity-defining regions (CDR) to retain functionally equivalent binding characteristics of an antibody that binds a desired cell surface receptor, and a binding fragment produced by phage display. Certain classes have subclasses as well, such as IgG1, IgG2, and others. Furthermore, in humans, the light chain can be a kappa chain or a lambda chain. Monoclonal antibodies are antibodies that are made by identical immune cells that are all clones of a unique parent cell. Monoclonal antibodies can have monovalent affinity, in that they bind to the same epitope. In contrast, polyclonal antibodies bind to multiple epitopes and are usually made by several different plasma cell lineages. In a preferred embodiment of the invention, the antibody is a monoclonal and/or a polyclonal antibody.

The ligands, preferably antibodies, preferably are conjugated with or coupled to a label to facilitate their detection. In one embodiment, those labels comprise radioactive, fluorescent, magnetic, luminescent, chemiluminescent, biological or enzymatic tags or labels of standard use in the art. In some embodiments, the label can be a small chemical molecule that is capable, acting alone, or in concert with other molecules or proteins, of providing a signal, that is detectable either directly or indirectly. A particularly preferred embodiment is the coupling of the ligand to a magnetic bead. Non-limiting examples of detectable labels that can be conjugated or coupled to polypeptides such as antibodies include but are not limited to radioactive labels, such as ³H, ¹¹C, ¹⁴C, ¹⁸F, ³²P, ³⁵S, ⁶⁴Cu, ⁷⁶Br, ⁸⁶Y, ⁹⁹Tc, ¹¹¹In, ¹²³I, ¹²⁵I, or ¹⁷⁷Lu, enzymes, such as horseradish peroxidase, fluorophores, chromophores, chemiluminescent agents, chelating complexes, dyes, colloidal gold or latex particles.

Ligands may, apart from antibodies, also be muteins or other binding molecules such as DARPins, nucleic acid aptamers, affimers, affibodies, affilins, affitins (nanofitins), alphabodies, anticalins, avimers, fynomers, Kunitz domain peptides or monobodies.

In another embodiment of the invention, the label can also be an enzyme that interacts with a substrate to produce the detectable signal; or a protein that is detectable by antibody binding or by binding to a suitably labeled ligand. A variety of enzyme systems operate to reveal a colorimetric signal in an assay, for example, glucose oxidase, horseradish peroxidase (HRP) or alkaline phosphatase (AP), and hexokinase in conjunction with glucose-6-phosphate dehydrogenase. Other label systems that can be used include nanoparticles or quantum dots.

The expression of the cell CD51, CD177 and/or CD275 may be detected with different methods. In one embodiment of the invention, flow cytometry, Western Blot, Northern Blot, microarray, polymerase chain reaction and/or quantitative polymerase chain reaction (qPCR) are used for the detection. In a preferred embodiment of the invention, flow cytometry is used for the detection of CD51, CD177 and/or CD275 expression and in another preferred embodiment of the invention qPCR is used for the detection of CD51, CD177 and/or CD275 expression.

As used herein, "quantitative polymerase chain reaction" (qPCR) or "Real-Time polymerase chain reaction" (Real-Time PCR) refer to a laboratory technique based on the polymerase chain reaction. It monitors the amplification of a targeted DNA molecule during the PCR, i.e. in real-time, and not at its end, as in conventional PCR. Real-time PCR can be used quantitatively (quantitative real-time PCR), and semi-quantitatively, i.e. above/below a certain amount of DNA molecules (semi quantitative real-time PCR). Two common methods for the detection of PCR products in real-time PCR are known to the skilled person: non-specific fluorescent dyes that intercalate with any double-stranded DNA or sequence-specific DNA probes consisting of oligonucleotides that are labelled with a fluorescent reporter which permits detection only after hybridization of the probe with its complementary sequence.

The expression of tissue-specific gene products can also be detected at the mRNA level by Northern blot analysis, dot-blot hybridization analysis, or by reverse transcriptase initiated polymerase chain reaction (RT-PCR) using sequence-specific primers in standard amplification methods.

The expression of a cell surface marker, i.e. staining intensity of cells, can be monitored by flow cytometry, where lasers detect the quantitative levels of fluorochrome (which is proportional to the amount of cell surface marker bound by specific reagents, e.g. antibodies). Although the absolute level of staining can differ with a particular fluorochrome and reagent preparation, the data can be normalized to a control. The readouts of selected parameters are capable of being read simultaneously, or in sequence during a single analysis, as for example through the use of fluorescent antibodies to cell surface molecules. As an example, these can be tagged with different fluorochromes, fluorescent bead, tags, e.g. quantum dots, etc.

In some embodiments, the expression levels of any marker in any PSC/iPSC/ESC-derived cell types or PSC/iPSC/ESC -derived cell populations is at least about 4-fold higher, at least about 6-fold higher, at least about 8-fold higher, at least about 10-fold higher, at least about 15-fold higher, at least about 20-fold higher, at least about 40-fold higher, at least about 80-fold higher, at least about 100-fold higher, at least about 150-fold higher, at least about 200-fold higher, at least about 500-fold higher, at least about 750-fold higher, at least about 1000-fold higher, at least about 2500-fold higher, at least about 5000-fold higher, at least about 7500-fold higher or at least about 10,000-fold higher as compared to a standardized or normalized control marker. Markers in this context not only comprise CD51, CD177 and/or CD275 but as well cell surface markers for cell populations as defined herein.

The cells used in accordance with the method of this invention PSC/iPSC/ESC or PSC/iPSC/ESC-derived cells can be obtained from any mammal. In some embodiments, cells are derived from cats, dogs, cows, pigs, horses and/or production animals. In a preferred embodiment, the cells are derived from a human.

Embodiments for the separating step according to the present invention include those described herein such as, antibody-coated magnetic beads (MACS), affinity chromatography and "panning" with antibody attached to a solid matrix or solid phase capture medium, e.g. plate, column or other convenient and available technique. Techniques providing accurate separation include flow cytometry methods which are useful for measuring cell surface and intracellular parameters, as well as shape change and granularity and for analyses of beads used as antibody- or probe-linked reagent. Using those data obtained with flow cytometry a person skilled in the art may identify subpopulations and may then isolate the specific subpopulations. Preferably, these data can be used to sort pancreatic progenitor cells out of PSC/iPSC/ESC-derived subpopulations on the basis of CD51, CD177 and/or CD275 expression. Uses of MACS and flow cytometry are exemplified in the examples.

In one embodiment of the invention described herein, affinity chromatography is used for the purification of PSC/iPSC/ESC-derived cells. Affinity chromatography is a method of separating biochemical mixtures based on a highly specific interaction such as that between antigen and antibody, enzyme and substrate, or receptor and ligand. It is a type of chromatographic lab technique used for purifying biological molecules or cell subpopulations within a mixture by exploiting molecular properties, e.g. the expression of a cell surface marker. Ligands, e.g. biological macromolecules such as antibodies or fragments thereof, enzymes and/or other proteins, interact with other molecules with high specificity through several different types of bonds and interaction. Such interactions comprise hydrogen bonding, ionic interaction, disulfide bridges, hydrophobic interaction, and more. The high selectivity of affinity chromatography is caused by allowing the desired molecule to interact with the stationary phase and become trapped within the column in order to be separated from the undesired material which will not interact and elute first. In a preferred embodiment of the invention, the separating step comprises affinity chromatography.

In a further embodiment of the invention, flow cytometry is used for the purification of PSC/iPSC/ESC-derived cell populations. In a preferred embodiment of the invention, the separating step comprises flow cytometry. In addition to the flow cytometry used for e.g. the detection expression of surface markers, it can be combined with a device that allows separating certain subpopulations and is often referred to as fluorescence-activated cell sorting (FACS). It provides a method for sorting a heterogeneous mixture of biological cells into two or more containers, one cell at a time, based upon the specific light scattering and fluorescent characteristics of each cell. It is a useful scientific instrument as it provides fast, objective and quantitative recording of fluorescent signals from individual cells as well as physical separation of cells of particular interest.

The cell suspension is entrained in the center of a narrow, rapidly flowing stream of liquid. The flow is arranged so that there is a large separation between cells relative to their diameter. A vibrating mechanism causes the stream of cells to break into individual droplets. The system is adjusted so that there is a low probability of more than one cell per droplet. Just before the stream breaks into droplets, the flow passes through a fluorescence measuring station where the fluorescent character of each cell of interest is measured. An electrical charging ring is placed just at the point where the stream breaks into droplets. A charge is placed on the ring based immediately prior to fluorescence intensity being measured, and the opposite charge is trapped on the droplet as it breaks from the stream. The charged droplets then fall through an electrostatic deflection system that diverts droplets into containers based upon their charge. In some systems, the charge is applied directly to the stream, and the droplet breaking off retains charge of the same sign as the stream. The stream is then returned to neutral after the droplet breaks off. For the detection of specific subpopulations, ligands, preferably antibodies comprise a label, preferably a fluorescent label as described herein. If each cell surface marker is labeled with a different label, it is possible to distinguish between different cell populations described herein and thereby enrich the desired subpopulation.

For the purpose of isolation of specific subpopulations of a cell population, magnetic cell sorting (MACS) may be used, summarized in the following embodiments: In one embodiment, the ligand is immobilized on a capture medium. The capture medium preferably comprises a bead and in an especially preferred embodiment, the bead is magnetic. In a preferred embodiment the ligand is conjugated to a magnetic bead.

In one embodiment, the ligand, agent, and/or antibodies described herein are directly or indirectly conjugated to a magnetic reagent. A "magnetic reagent" is a particle capable of reacting with a ligand via direct/covalent or indirect coupling. The term "magnetic" in "magnetic reagent" as used herein refers to all subtypes of magnetic particles, which can be prepared with methods well known to the skilled person in the art, especially ferromagnetic particles, superparamagnetic particles and paramagnetic particles. Preferably, the magnetic reagent is a magnetic bead. Direct conjugation to a magnetic reagent is achieved by use of various chemical linking groups, as known in the art. In some embodiments, the antibody is coupled to the magnetic reagent through side chain amino or sulfhydryl groups and heterofunctional cross-linking reagents. A large number of heterofunctional compounds are available for linking to entities. For example, at least, 3-(2-pyridyidithio)propionic acid N-hydroxysuccinimide ester (SPDP) or 4-(N-maleimidomethyl)-cyclohexane-I-carboxylic acid N-hydroxysuccinimide ester (SMCC) with a reactive sulfhydryl group on the antibody and a reactive amino group on the magnetic particle can be used. An example of a magnetic separation device is described in WO 90/07380, PCT/US96/00953, and EP 438520. The purified cell population can be collected in any appropriate medium, preferably StemMACS IPS Brew (Miltenyi-Biotec). The pancreatic progenitor cells may then be seeded in StemMACS IPS-Brew medium for further differentiation.

In one embodiment of the invention magnetic cell sorting is used for the separation of subpopulations of a cell population. Methods to separate cells magnetically are commercially available e.g. from Invitrogen, Stem cell Technologies, in Cellpro, Seattle or Advanced Magnetics, Boston. For example, monoclonal antibodies can be directly coupled to magnetic polystyrene particles like Dynal M 450 or similar magnetic particles and used e.g. for cell separation. The Dynabeads technology is not column based, instead these magnetic beads with attached cells enjoy liquid phase kinetics in a sample tube, and the cells are isolated by placing the tube on a magnetic rack. However, in a preferred embodiment for enriching, sorting and/or detecting cells from a sample monoclonal antibodies or antigen binding fragments thereof are used in conjunction with colloidal superparamagnetic microparticles having an organic coating by e.g. polysaccharides (Magnetic-activated cell sorting (MACS^{®}) technology (Miltenyi Biotec, Bergisch Gladbach, Germany)). These particles (nanobeads or MicroBeads) can be either directly conjugated to monoclonal antibodies or antigen binding fragments thereof or used in combination with anti- immunoglobulin, avidin or anti-hapten-specific MicroBeads. The MACS^{®} technology allows cells to be separated by incubating them with magnetic nanoparticles coated with antibodies or antigen binding fragments thereof directed against a particular surface antigen. This causes the cells expressing this antigen to attach to the magnetic nanoparticles. Afterwards the cell solution is transferred on a column placed in a strong magnetic field. In this step, the cells (expressing the antigen) attach to the nanoparticles and stay on the column, while other cells (not expressing the antigen) flow through. With this method, the cells can be separated positively or negatively with respect to the particular antigen(s). In case of a positive selection the cells expressing the antigen(s) of interest, which attached to the magnetic column, are washed out to a separate vessel, after removing the column from the magnetic field. In case of a negative selection the antibody used is directed against surface antigen(s), which are known to be present on cells that are not of interest. After application of the cells/magnetic nanoparticles solution onto the column the cells expressing these antigens bind to the column and the fraction that goes through is collected, as it contains the cells of interest. As these cells are non-labeled by an antibody coupled to nanoparticles, they are "untouched". The procedure can be performed using direct magnetic labeling or indirect magnetic labeling. For direct labeling the specific antibody is directly coupled to the magnetic particle. Indirect labeling is a convenient alternative when direct magnetic labeling is not possible or not desired. A primary antibody, a specific monoclonal or polyclonal antibody, a combination of primary antibodies, directed against any cell surface marker can be used for this labeling strategy. The primary antibody can either be unconjugated, biotinylated, or fluorophore-conjugated. The magnetic labeling is then achieved with anti-immunoglobulin MicroBeads, anti-biotin MicroBeads, or anti-fluorophore MicroBeads. The above-described processes can also be performed in a closed system such as CliniMACS^{®} (Miltenyi Biotec GmbH, Germany) or CliniMACS^{®} Prodigy (Miltenyi Biotec GmbH, Germany).

The term "closed system" as used herein refers to any closed system which reduces the risk of
cell culture contamination while performing culturing processes such as the introduction of new material and performing cell culturing steps such as proliferation, differentiation, activation, genetic modification and/or separation of cells. Such a system allows operating under GMP or GMP-like conditions ("sterile") resulting in cell compositions which are clinically applicable. An example for a closed system is the CliniMACS Prodigy^{®} (Miltenyi Biotec GmbH, Germany, WO2009/072003).

The terms "automated method" or "automated process" as used herein refer to any process being automated through the use of devices and/or computers and computer software which otherwise would or could be performed manually by an operator. Methods (processes) that have been automated require less human intervention and less human time to deliver. In some instances a method is automated if at least one step of the method is performed without any human support or intervention. Preferentially the method is automated if all steps of the method are performed without human support or intervention.

The method of purifying pancreatic progenitor cells in accordance with the present invention results in one embodiment of the present invention in a cell composition comprising at least 10%, at least 20% at least 30%, at least 40%, at least 50%, at least 60%, at least 70%, at least 80%, at least 90% pancreatic progenitor cells, which are positive for CD51 and/or CD177. In a preferred embodiment, the composition comprises at least 50 % CD177-positive cells. This composition can be subsequently used for generation of pancreatic beta cells. Preferably, the cells comprised in the composition, preferably pancreatic progenitor cells, are human. In a further embodiment, the composition comprises at least 10%, at least 20% at least 30%, at least 40%, at least 50%, at least 60%, at least 70%, at least 80%, at least 90% CD275-negative pancreatic progenitor cells. In a preferred embodiment, the composition comprises at least 50% pancreatic progenitor cells, which are negative for CD275. Preferably, the cells comprised in the composition are human.

In another aspect of the disclosure, the composition of pancreatic progenitor cells comprise at least 10%, at least 20% at least 30%, at least 40%, at least 50%, at least 60%, at least 70%, at least 80%, at least 90% CD275-positive cells. Within this specific aspect of the disclosure, sorted for liver-specific cell surface marker CD275, pancreatic progenitor cells have to be rather seen as liver progenitor cells. Therefore, such a composition can be used for generating cells of the liver lineage. Accordingly, also disclosed but not part of the invention is a method of purifying a liver progenitor cell comprising:
(a) exposing a population of cells derived from pluripotent stem cells, induced pluripotent stem cells and/or embryonic stem cells comprising a liver progenitor cell, to a ligand which binds to a cell surface marker on the liver progenitor cell, wherein the cell surface marker is CD275 and
(b) separating the liver progenitor cell from cells derived from pluripotent stem cells, induced pluripotent stem cells and/or embryonic stem cells which do not bind to the ligand, thereby purifying said liver progenitor cell.

The method of purifying liver progenitor cells comprises preferably a further step, i.e. the step of exposing said population of cells to a ligand which binds to CD51 and/or CD177.

Preferably, the ligand that is binding to CD51, CD177 and/or CD275 used for purifying liver progenitor cells in accordance with the method of the present disclosure is an antibody or binding fragment thereof.

The antibody or antibody fragment that is binding to CD51, CD177 and/or CD275 used in accordance with the method of purifying liver progenitor cells preferably is a monoclonal antibody or a polyclonal antibody.

Preferably, the ligand, which is binding to CD51, CD177 and/or CD275 and used for purifying liver progenitor cells in accordance with the method of the present disclosure, is conjugated with a label.

Preferably, the expression of the cell surface markers CD51, CD177 and/or CD275 is detected by quantitative polymerase chain reaction (qPCR) and/or flow cytometry in accordance with the method of purifying liver progenitor cells described herein.

The separation step of the method of purifying liver progenitor cells preferably comprises affinity chromatography and/or flow cytometry.

The liver progenitor cells purified, which are not part of the invention, preferably are human.

The ligand used in the method in accordance to the method of purifying liver progenitor cells or the composition of liver progenitor cells described herein preferably is immobilized on a capture medium. The capture medium preferably comprises a bead and/or the ligand preferably is conjugated to a magnetic reagent.

The method of purifying liver progenitor cells in accordance with the present disclosure preferably results in a cell composition comprising at least 50% liver progenitor cells.

A composition purified according to the method of purifying liver progenitor cells preferably comprises at least 50% liver progenitor cells which are positive for CD275. Preferably the liver progenitor cells of the composition described herein are bound to a ligand that binds CD275 on the liver progenitor cells. The composition of liver progenitor cells purified in accordance to the method of the present disclosure and/or the liver progenitor cells of the composition bound to a ligand that binds CD275 on the liver progenitor cells preferably are human.

Further, the present disclosure relates to the use of a ligand which binds to a cell surface marker on a liver progenitor cell, wherein the cell surface marker is CD275, for purifying a liver progenitor cell from a population of cells derived from pluripotent stem cells, induced pluripotent stem cells and/or embryonic stem cells.

The present disclosure also relates to a kit comprising a ligand which binds to CD275 and means for differentiating pluripotent stem cells, induced pluripotent stem cells and/or embryonic stem cells into a population of liver progenitor cells.

In a preferred embodiment of the present invention, the pancreatic progenitor cells comprised in compositions created by methods of the present invention are bound to a ligand that binds CD177 on the cell surface. In a more preferred embodiment, the cells comprised in the composition are human.

The present disclosure relates in a further aspect to the use of a ligand, which binds to a cell surface marker, for purifying pancreatic progenitor cells from a population and/or composition derived from PSC/iPSC/ESC. The surface marker is CD177. A preferred embodiment refers to the use of a ligand, which binds to a cell surface marker on a pancreatic progenitor cell, wherein the cell surface marker is CD177, for purifying a pancreatic progenitor cell from a population of cells derived from PSC/iPSC/ESC, wherein the pancreatic progenitor cell is sorted at the stage of the definitive endoderm (DE), the anterior definitive endoderm (ADE), the posterior gut tube (PGT) and/or the ventral foregut (VFG).

In further embodiments, the present invention relates to the use of a ligand, which binds to a cell surface marker, wherein the cell surface marker is CD177, for purifying pancreatic progenitor cells, comprising the steps of
(a) exposing a population of cells derived from pluripotent stem cells, induced pluripotent stem cells and/or embryonic stem cells comprising a pancreatic progenitor cell, to a ligand which binds to a cell surface marker on the pancreatic progenitor cell, wherein the cell surface marker is CD177 and
(b) separating the pancreatic progenitor cell from cells derived from pluripotent stem cells, induced pluripotent stem cells and/or embryonic stem cells which do not bind to the ligand, thereby purifying said pancreatic progenitor cell,
wherein the pancreatic progenitor cell is sorted at the stage of the definitive endoderm (DE), the anterior definitive endoderm (ADE), the posterior gut tube (PGT) and/or the ventral foregut (VFG),wherein the pancreatic progenitor cell preferably comprises CD177 on the surface.

### Kits, which are not part of the invention

This disclosure also relates to a kit comprising ligands, which bind CD177, and means for differentiating pluripotent stem cells (PSC), induced pluripotent stem cells (iPSC) and/or embryonic stem cells (ESC) into a population of definitive endoderm (DE), pancreatic progenitor cells (PP1 and/or PP2) and/or pancreatic beta-cells capable of producing insulin in a glucose concentration-dependent manner.

"Means for differentiating" or "means for differentiation" as used herein, comprise different cell media - basal media or supplemented media - capable of inducing a differentiation of pluripotent stem cells (PSC), induced pluripotent stem cells (iPSC) and/or embryonic stem cells (ESC) into a population DE, PP and/or pancreatic beta-cells capable of producing insulin in a glucose concentration-dependent manner.

Herein, a "basal medium" refers to a solution of amino acids, vitamins, salts, and nutrients that is effective to support the growth of cells in culture, although normally these compounds will not support cell growth unless supplemented with additional compounds. The nutrients comprise a carbon source, preferably sugars, more preferably glucose that can be metabolized by the cells, as well as other compounds necessary for the cells to survive. These are compounds that the cells themselves cannot synthesize. Many different basal media are known to the skilled person. Preferred basal media referred to by this disclosure are MCDB 131 medium, IPS-Brew medium and/or BLAR medium.

In preferred aspects of the disclosure, the basal medium is supplemented with different growth and differentiation stimulating factors to form a "supplemented medium". Various supplements are known to the skilled artisan. Preferred supplements are listed in the following table 1:

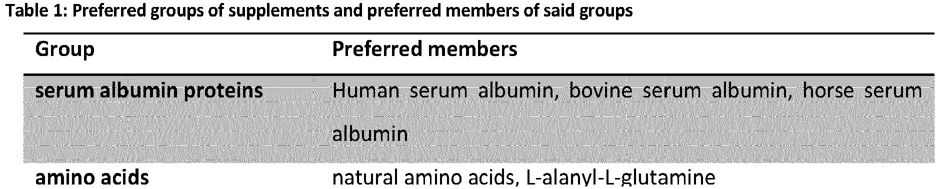

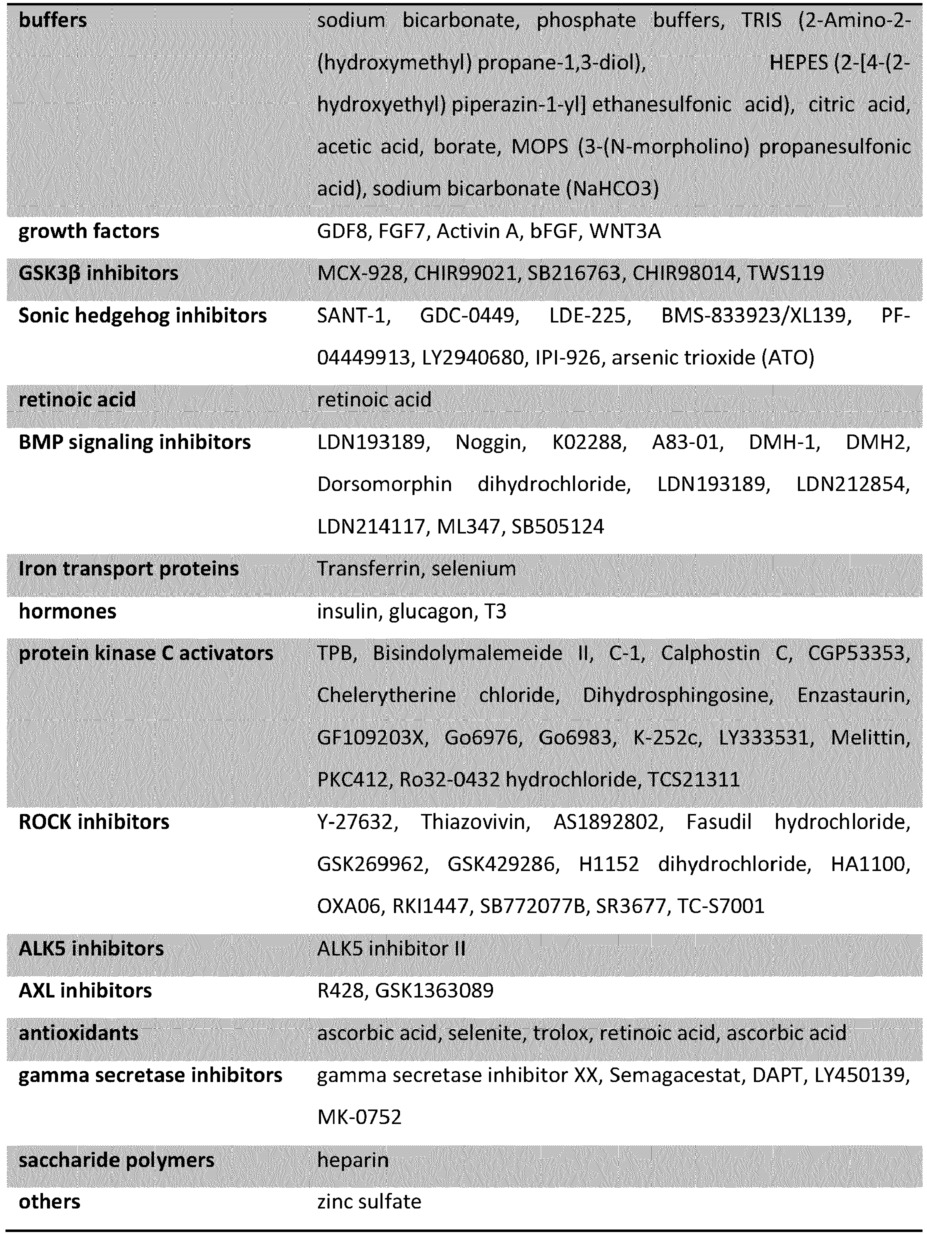

Supplemented Media can be generated by the combination of a basal medium and one and/or more supplements known to the skilled artisan, preferably those in table 1. In additional aspects of the disclosure, premixed supplements for the addition to a basal medium may be used. A further aspect of the disclosure comprises a convenience blend or supplemental ingredients in a dried powder form that can form a supplemented medium by the addition of a liquid, preferably water. A kit according to this disclosure may in certain embodiments comprise a basal medium, a supplemented medium and/or premixed supplements.

In aspects of the disclosure a kit contains the components taught above, e.g., at least one soluble ligand that binds a cellular target in the sample; at least one soluble ligand that binds a soluble analyte in the sample or at least one competing soluble analyte (preferably labeled); and a solid phase capture medium that binds directly to the soluble analyte, indirectly to the soluble analyte, or to the soluble ligand that binds to the soluble analyte. The kits also include instructions for performing the particular assay, various diluents and buffers, and signal-generating reagents, such as fluorophores, enzyme substrates, cofactors and chromogens. Other components can include indicator charts for colorimetric comparisons, disposable gloves, decontamination instructions, applicator sticks or containers, and a sample preparatory cup.

In another aspect of the disclosure, the cell medium capable of inducing a differentiation of pluripotent stem cells (PSC), induced pluripotent stem cells (iPSC) and/or embryonic stem cells (ESC) into a population DE, PP and/or pancreatic beta-cells capable of producing insulin in a glucose concentration-dependent manner is the StemMACS IPS-Brew medium (Miltenyi-Biotec). With this cell medium, which is supplemented with cytokines for pancreatic β-cell differentiation, PSC/iPSC/ESC may be differentiated using an embryoid body format.

In one aspect of the disclosure, a kit useful for the performance of the above-described immune assays include, as a component, a solid matrix or a solid phase capture medium associated with multiple first ligands that bind the cellular marker or target and is associated with a first detectable label. The kit further comprises a second, third and/or fourth ligand that is capable of binding to a second, third and/or fourth cellular marker or target and or soluble analyze. The second, third and/or fourth ligands are associated with a second, third and/or fourth detectable label.

Such kits are useful for evaluating PSC/iPSC/ESC populations and/or PSC/iPSC/ESC-derived cell population samples for purposes of enriching, isolating, depleting, separating, sorting, purifying and/or determining levels of certain cell types or bound components or soluble antigens or analytes thereof. Such a diagnostic kit contains the dyes, ligands, capture medium, and other components of the aspects of the disclosure described herein. Such kits also contain labels, exemplified above, pre-attached to the other components of the specific assay to be performed, or provided separately for attachment to a selected component, e.g., a substrate. Alternatively, such kits can contain a simple mixture of such compositions or means for preparing a simple mixture.

### REFERENCES

D'Amour KA, Agulnick AD, Eliazer S, Kelly OG, Kroon E, Baetge EE (2005): Efficient differentiation of human embryonic stem cells to definitive endoderm. Nature Biotechnology, Vol. 23, pp. 1534-1541.
D'Amour KA, Bang AG, Eliazer S, Kelly OG, Agulnick AD, Smart NG, Moorman MA, Kroon E, Carpenter MK, Baetge EE (2006): Production of pancreatic hormone-expressing endocrine cells from human embryonic stem cells. Nature Biotechnology, Vol. 24, pp. 1392-1401.
Köhler G, Milstein C (1975): Continuous cultures of fused cells secreting antibody of predefined specificity. Nature, Vol. 256, pp. 495-497
Kroon E, Martinson LA, Kadoya K, Bang AG, Kelly OG, Eliazer S, Young H, Richardson M, Smart NG, Cunningham J, Agulnick AD, D'Amour KA, Carpenter MK, Baetge EE (2008): Pancreatic endoderm derived from human embryonic stem cells generates glucose-responsive insulin-secreting cells in vivo. Nature Biotechnology, Vol. 26(4), pp. 443-452.
Pagliuca FW, Millman JR, Gürtler M, Segel M, Van Dervort A, Ryu JH, Peterson QP, Greiner D, Melton DA (2014): Generation of Functional Human Pancreatic β Cells In Vitro. Cell, Vol. 159, pp. 428-439.
Rezania, A. et al. (2013): Enrichment of human embryonic stem cell-derived NKX6.1-expressing pancreatic progenitor cells accelerates the maturation of insulin-secreting cells in vivo. STEM CELLS 31, 2432-2442.
Rezania A, Bruin JE, Arora P, Allison R, Batushansky I, Asadi A, O'Dwyer S, Quiskamp N, Mojibian M, Albrecht T, Yang YHC, Johnes JD, Kieffer TJ (2014): Reversal of diabetes with insulin-producing cells derived in vitro from human pluripotent stem cells. Nature Biotechnology, Vol. 32(1), pp. 1121-1133.
Takahashi K, Yamanaka S (2006): Induction of Pluripotent Stem Cells from Mouse Embryonic and Adult Fibroblast Cultures by Defined Factors. Cell, Vol. 126(4), pp. 663-676.

### REFERENCE EXAMPLES

The following examples illustrate the invention. These examples should not be construed as to limit the scope of the invention. The examples are included for purposes of illustration and the present invention is limited only by the claims.

### Materials and methods

Unless specified otherwise, the following methods and materials were used:

### Cell sources

Human islets were obtained from the Rudbecklaboratoriet C11 (Uppsala, Sweden) with informed consent. The H9 hESC line was obtained from WiCell Research Institute, Inc. (Madison, Wl). The Mel1-NKX6.1-GFP and Mel1- INS-GFP were obtained from Australian Stem Cell Centre (Clayton, Victoria). An episomal reprogrammed iPSC line was generated in lab from control group of MODY-4 patients (Gibco Human Episomal iPSC, Cat # A18945, Life Technologies, CA). Both cell lines have been authenticated by Cell Line Genetics (Madison, WI) and confirmed to be mycoplasma-free by using the Lonza MycoAlert Mycoplasma Detection Kit (Lonza, Cat# LT07-418). The hES cell lines were used under the permission of Robert Koch Institute.

### In vitro differentiation of human pluripotent stem cells towards pancreatic β-like cells.

H9, Mel1-NKX6.1-GFPand iPSCs were cultured on 1:30 diluted Geltrex (Invitogen, U.K, Cat#A1413302) in StemMACS iPS-Brew medium (Miltenyi Biotech,Germany, Cat#130-104-368). At ^{∼}70-80% confluency, cultures were rinsed with 1× DPBS without Mg₂₊ and Ca₂₊ (Invitrogen, Cat#14190) followed by incubation with 0.5 mM EDTA (Applichem, Cat#12604-021) for 2-3 min at 37 °C. Single cells were rinsed with iPS-Brew, and spun at 1,300 r.p.m. for 3 mins. The resulting cell pellet was suspended in iPS-Brew medium supplemented with Y-27632 (10 µM; Sigma-Aldrich; MO, Cat#Y0503) and the single cell suspension was seeded at ^{~}1.5-2 × 10s cells/cmz on Geltrex-coated surfaces. Cultures were fed every day with iPS-Brew medium and differentiation was initiated 24 h following seeding, resulting in ^{~}90% starting confluency. The cells were differentiated towards definitive endoderm using MCDB131 medium supplemented with 0.5% BSA (Sigma, Cat#10775835001), 100 ng/ml Activin A (Peprotech, Cat#120-14-300) and 25 ng/ml WNT3A (Peprotech, Cat#315-20) for the first day. For the next 2 days, the cells were treated with MCDB131 supplemented with 0.5% BSA and 100ng/ml Activin A. For differentiation towards β cells, Rezania, et.al; 2014 β- cell differentiation protocol was used 14. In nutshell, the cells were differentiated towards primitive gut tube with MCDB131 supplemented with 0.5 % BSA, 50 ng/ml of FGF7 (Peprotech, Cat#100-19-100) and 0.25 mM Vit.C (Sigma, Cat#120-14-300) for 2 days. For differentiation towards posterior foregut, the cells were further exposed to MCDB131 medium supplemented with 1xGlutamax (Gibco, Cat#A12860-01), 2 % BSA (Cat#10775835001), 0.25 mM Vit. C (Sigma, Cat#A4544-25G), 50 ng/ml FGF7, 0.25 µM SANT-1 (Sigma, Cat#S4572-5MG), 1 µM retinoic acid (Sigma, Cat#R2625-50MG), 100 nM LDN193189 (Sigma, Cat#04-0074), 1:200 ITS-X (Gibco, Cat#51500-056) and 200 nM TPB (Merk Millipore, Cat#565740-1MG) for 2 days. For the WNT inhibition experiment only, 1.25 µM of IWP2 (Tocris, Cat#3533-10) was added to the cultures. The cells were then further differentiated towards pancreatic endoderm using MCDB131 supplemented with 1xGlutamax, 10 mM final glucose concentration, 2% BSA, 0.25 mM Vit.C, 2 ng/ml FGF7, 0.25 µM SANT-1, 0.1 µM retinoic acid, 200 nM LDN193189, 1:200 ITS-X and 100 nM TPB for 3 days. For induction of pancreatic endocrine precursors, the cells were next exposed to MCDB131 medium supplemented with 1xGlutamax, 20 mM final glucose concentration, 2 % BSA, 0.25 µM SANT-1, 0.05 µM retinoic acid, 100 nM LDN193189, 1:200 ITS-X, 1 µM T3 (Sigma, Cat#T6397-100MG), 10 µM ALK5 Inhibitor II (Enzo life sciences, Cat#ALX-270-445-M005), 10 µM zinc sulphate (Sigma, Cat#SI Z0251-100G) and 10 µg/ml heparin (Sigma, Cat#H3149) for 3 days. Hormone positive cells were generated by exposing the endocrine progenitors from last step with MCDB131 supplemented with 1xGlutamax, 20 mM final glucose concentration, 2% BSA, 100 nM LDN193189, 1:200 ITS-X, 1µM T3, 10 mM ALK5 Inhibitor II, 10 µM zincsulphate, 100 nM gamma secretase inhibitor XX (Merck, Cat#565789) for the first 7 days only and 10 µg/ml of heparin for 7-15 days. For maturation of β-like cells, the cells from previous stage were treated with 2% BSA, 1:200 ITS-X, 1 µM T3, 10 µM ALK5 inhibitor II, 10 µM zinc sulphate, 1 mM N-acetyl cysteine (Sigma, Cat#A9165), 10 µM Trolox (EMD, Cat#648471), 2 µM R428 (SelleckChem, Cat# S2841) and 10 mg/ml of heparin for 15 days.

### Sorting ADE subpopulations

On day 4 of differentiation (DE/ADE), the differentiated samples were collected and stained for surface markers. For staining of the surface markers, 10µl Ab (conjugated to APC) was added per 1×10₆ cells in 100µl volume of MCDB1 + 0.5% BSA. The cells were stained in dark for 15 mins on ice. For magnetic labelling of the antibody the stained cells were washed 3x PBS to remove the antibody and then suspended in 80 ml of MCDB131+0.5% BSA medium with 20 µLs of Anti-APC Microbeads per 10 Mio of total cells. The cells were incubated for 15 mins at 37°C. The cells were washed with 1-2 ml of PBS and then suspended up to 20×10₆ cells in 500 µL of MCDB131+0.5% BSA and proceed with magnetic sorting. For Magnetic sorting of the ADE subpopulations, the LS column was placed in magnetic sorter. The column was rinsed with 3 ml of PBS. Cell suspension was applied to the column and the flow through containing unlabelled cells was discarded. The column was then washed 3x with 3 ml of PBS. The antibody positive cells were collected by removing the column from the separator and flushing it with 5 ml of medium. The cells were then seeded in iPS-Brew medium supplemented with 10 µM Y-compound at the seeding density of 2-10×10₃ cells in 1 well of ultra-low attachment round bottom 96 well plates to form an aggregate or 4x10s cells in one well of IBID chamber for further differentiation and staining.

### Quantitative qPCR analysis and gene profiling

Gene expression was assessed in differentiated cells by Taqman Arrays (Applied Biosystems). Data were analysed using Expression Suite Software (Applied Biosystems) and normalized to undifferentiated hESCs using ΔΔCt method.

### Immunofluorescence staining and Western Blotting

For immunocytochemistry, the cells or aggregates were fixed in 4% PFA. The cells were then permeabilised using 0.5% Saponin in blocking buffer. For Western Blotting, cells were dissociated in RIPA buffer directly after sorting. Cell lysates were resolved by SDS-PAGE, transferred to PVDF membrane (Biorad) and incubated with the antibodies. Protein bands were visualized using HRP conjugated antibodies and chemiluminescence reagent (Millipore). The bands were quantified with ImageJ.

### Flow cytometry analysis

hESCs and differentiated cells were dissociated and single cell suspension was prepared. The cells were fixed, permeablilised and stained for DE, pancreatic endoderm, pancreatic progenitors and hormone positive markers. FACS gating was determined using isotype antibodies.

### Static glucose stimulation insulin secretion

Static glucose stimulated insulin secretion (GSIS) of the generated β-like cells was performed based on previous described protocols 14,56. Briefly, 5 aggregates (1,00,000 cells in total) were picked and rinsed three times with KRBH buffer (129 mM NaCl, 4.8 mM KCI, 2.5 mM CaCl₂, 1.2 mM MgSOz, 1 mM Na₂HPO₄, 1.2 mM KH₂PO₄, 5 mM NaHCOs, 10 mM HEPES and 0.1% BSA in deionized water and sterile filtered) and then equilibrated in KRBH buffer at 37°C for 1 hr. Aggregates then were incubated in KRBH buffer spiked with 2.8 mM glucose for 60 mins at R.T. Supernatants were collected and the aggregates were transferred to KRBH buffer spiked with 16.7 mM glucose for 60 mins. Supernatants were collected again. At the end of the experiment, cell aggregates were dissociated into single cells and the cell numbers were counted to normalize the GSIS. Mercodia Human Insulin ELISA kit (Mercodia, Cat# 10-1113-01) was used to measure the insulin content in supernatant sample following manufacturer's protocol.

### Affymetrix microarray analysis

For gene profiling, total RNA was extracted using miRNeasy Mini kit (Qiagen, Cat#217004), RNA integrity was checked using Agilent 2100 Bioanalyzer (Agilent RNA 6000 Pico Kit) and only high quality RNA (RIN>7) was used for microarray analysis. Total RNA (5 ng) was amplified using the Ovation Pico WTA System V2 in combination with the Encore Biotin Module (Nugen). Amplified cDNA was hybridised on Affymetrix Human Gene ST 2.0 arrays (Affymetrix, 902113). Staining and scanning (GeneChip Scanner 3000 7G) was done according to the Affymetrix expression protocol including minor modifications as suggested in the Encore Biotion protocol. Expression console (v.1.4.1.46, Affymetrix) was used for quality control. All subsequent computational analysis was performed in R using Bioconductor packages. Expression data were RMA normalised using the oligo package (version 1.42.0) and probe sets were annotated using the package hugene20sttranscriptcluster.db (version 8.7.0). Differential expression analyses were performed using the limma package (version 3.34.9) and P-values were adjusted for multiple testing by Benjamini-Hochberg correction. A gene was considered as differentially expressedif the raw p-value was below a threshold of 0.01 and the fold-change was greater than or equal to 1.5. Functional enrichments were conducted using HOMER₅₇. All microarray data is available at GEO under the accession number GSE113791 (https://www.ncbi.nlm.nih.gov/geo/query/acc.cgi?acc=GSE113791).

### Image analysis

Images were acquired with Leica SP5 confocal microscope and Zeiss LSM 880 Airy Scan confocal microscope. Images taken by Leica confocal were analysed using Leica LAS AF Lite. Images taken by Zeiss confocal microscope were analysed using Zeiss Zen Blue software.

### Statistical analysis

All values are depicted as means ± S.D. unless otherwise specified from atleast three independent experiments. Statistical significance is defined as P < 0.05. Sample sizes are provided in the figure legends. All statistics were performed using GraphPad Prism software 7 (GraphPad Software Inc., La Jolla, CA). Values were compared using unpaired or paired ttests as indicated for each experiment. Briefly, analysis of gene expression throughout differentiation (S1-S7) was performed using unpaired t-tests. Hormone content was compared between CD177 derived β-like cells and CXCR4 derived β-like cells using an unpaired, two-tailed t-test. All qPCR data were compared by an unpaired twotailed t-test. Comparison between expression levels of PDX1+ cells derived from CXCR4, CD177 and CD275 ADE cells was done using one way ANOVA. Investigators were not blinded to the treatment groups in our studies and no statistical test was used to determine sample size.

### Reference Example 1: Identification of novel surface marker antibodies that enrich for different definitive endoderm

The main hypothesis of the project was to find novel surface markers that can enrich different subpopulations in the definitive endoderm (DE). It is known in vivo that DE is patterned with gradient signals of TGFβ and Wnt3A and this patterning then in turn gives rise to various organs. The first objective was to figure out if this patterning effect is present in vitro too. For first experiments, FOXA2 and CXCR4, two known DE markers were used to assess the process of differentiation.

For generation of DE in vitro, the hESCs were cultured on geltrex in feeder free conditions in IPS-Brew medium (pluripotency medium) until confluency. Next, the medium was changed to differentiation medium (MCDB1+ 1x glutamax +0.5% BSA) supplemented with 20 ng/ml of Wnt3a and 100ng/ml of AA on day 1. From day2-day4, the cells were treated with differentiation medium supplemented with 100ng/ml AA. The differentiation scheme is shown in Fig. 1A.

Next day, the cells were trypsinized with EDTA and put on the Miltenyi plates at the seeding density of 200,000 cells/well to first stain with surface antibodies and CXCR4 for 15 minutes in dark at RT.

After 15 minutes, the cells were washed with PBS and then fixed with 4% PFA for intracellular FOXA2 staining. The cells were then permeabilized with 0.5% TritonX in 30 minutes along with blocking solution and then primary FOXA2 antibodies was added to the cells for 1 hour. Secondary antibody staining was carried out next for 30 minutes and the cells were then washed and FACS analysis was carried out using the MACS Quant of Miltenyi Biotec. Fig. 1B shows the screening setup for identification of novel surface markers that can enrich different DE subpopulations against known DE markers FOXA2 and CXCR4. Two markers - CD177 and CD275 - were identified as markers for distinct DE subpopulations (Fig. 1C).

To check the distribution of the novel antibodies in the DE, the hESCs were first differentiated towards DE using the above mentioned protocol and then the cells were stained for CD177 or CD275 along with FOXA2 and SOX17 or CXCR4 (FOXA2, SOX17 and CXCR4 all mark DE). Quantification of the cells marked by CD177 or CD275 in DE was done using FACS. The results indicate that CD177 and CD275 are different ADE subpopulations in both CXCR4+ and FOXA2+/SOX17+ DE populations (Fig. 1D).

For PCA (principal component analysis), the hESCs were first differentiated towards DE for 4 days using the above mentioned differentiation protocol and the cells were then stained with CD177, CD275 and CXCR4 (control) and sorted using magnetic labelling (Miltenyi Biotech) RNA was isolated from the cells using the manufacturer's protocol. Microarray analysis was done at Helmholtz Zentrum using Affymetrix arrays. PCA analysis reveals significant molecular differences between CD177, CXCR4 and CD275 subpopulations (Fig. 1E). This is further evident on GO analysis where they also show differential expression of the signaling pathways (Fig 1F).

### Reference Example 2: Non-canonical WNT/PCP pathway is differentially regulated in CD177⁺ subpopulation

For further characterization of the DE subpopulations, the hESC cells were differentiated towards DE and then MACS sorted the cells after staining them with CD177, CD275 and CXCR4. These cells were then subjected to microarray, qPCR quantification and western blotting to figure out specific differences in the subpopulations. A scheme showing differentiation and sorting is shown in Fig. 2A.

Definitive endoderm markers FOXA2 and SOX17 were first checked on total RNA levels. No differences in the levels of FOXA2 and SOX17 in between sub-populations were observed. Next, anterior definitive endoderm markers (ADE) such as Cerberus1 (CER1) and HHEX (hematopoietically-expressed homeobox protein) were analyzed. In vivo, the DE endoderm formed is then patterned into anterior side and the posterior side. The anterior side gives rise to organs such as lung, liver and pancreas while the posterior side gives rise to intestine. Since pancreas is more interesting in the context of this invention, it was just checked for the ADE markers CER1 and HHEX. It was observed that CD177+ DE enriches for CER1 subpopulation while CD275+DE enriches for HHEX subpopulation while the CXCR4+ DE marks the entire DE (Fig. 2B).

Next, different signaling pathways differentially regulated in these subpopulations were analyzed based on a microarray. It was found that the target genes of WNT/PCP pathway such as DVL2, ROR2, and JNK were all highly expressed in CD177+ DE subpopulation in the microarray when compared to CD275+ DE and CXCR4+ DE subpopulations (Fig. 2C). Gene ontology (GO) and differential gene expression analysis in three-way comparisons of the MACS-enriched CD177+, CD275+ and CXCR4+ populations showed enrichment for genes related to pathways that are involved in regulating endoderm patterning and proliferation as well as pancreatic cell-fate specification (Fig. 7A-D).These included positive regulation of TGF-β downstream signalling of activated FGFR1 pathway, positive regulation of MAPK cascades, retinoic acid (RA)-mediated signaling and regulation of canonical and non-canonical WNT/planar cell polarity (PCP) signalling. qPCR quantification of these genes showed significant expression of all the WNT/PCP signaling components such as DVL2, ROR2, WNTSa and WNT4 in CD177+ DE compared to CXCR4+ DE and CD275+ DE indicating an active WNT/PCP signaling in CD177+ DE (Fig. 2D, 2E). The phosphorylation of JNK, a target of WNT/PCP signaling in CD177+ DE confirms the activation of WNT/PCP pathway in these cells (Fig. 2F and Fig. 2G). In contrast, the expression of canonical ligand *WNT3A* and its target gene *AXIN2* were upregulated in the CD275+ ADE subpopulation (Fig. 7E). Nuclear translocation of β-catenin is a hallmark of canonical WNT activation, thus the inventors tested for the distribution of β-catenin in the nucleus and cytoplasm by immunofluorescence and nuclear fractionation in the isolated subpopulations. β-catenin accumulation was observed in the nuclear fraction of CD275+ and CXCR4+ populations (Fig. 7F) with clear signs of nuclear accumulation in immunofluorescence staining in the CD275+ ADE subpopulation (Fig. 4D). These results suggest that CD275+ ADE progenitors receive canonical WNT-β-catenin signalling and might be specified towards the liver fate, while CD177+ ADE progenitors receive non-canonical WNT/PCP signalling and might be specified towards the pancreatic fate.

### Reference Example 3: Enrichment of subpopulations can distinguish ventral and dorsal pancreatic progenitors

As WNT/PCP pathway is known to play a major role during pancreatic development, it was decided to look for the differential potential of these different DE subpopulations at various stages of pancreatic development in vitro. For this, the hESCs were differentiated towards DE and then sorted for CD177⁺, CD275⁺ and CXCR4⁺ DE subpopulations using the novel antibodies. These subpopulations were then further differentiated first into primitive gut tube and then into pancreatic progenitors (PP) using the Rezania et.al, 2014 protocol. The experimental scheme is depicted in Fig. 3A.

In vivo, during the process of pancreatic development, the primitive gut tube gives rise to ventral foregut endoderm and dorsal foregut endoderm. The ventral foregut endoderm gives rise to both liver and pancreatic progenitors while the dorsal foregut endoderm gives rise only to the pancreatic progenitors. SOX17 is the marker for ventral foregut endoderm. It was observed that in CD177⁺ cells, the expression of SOX17 peaks during DE stage and drops as the cells enter primitive gut tube. Interestingly, in CD177⁺ cells the expression of SOX17 declines progressively as the differentiation proceeds towards pancreatic progenitor stage. However, in CD275⁺ and CXCR4⁺ cells after the initial peaking in the DE stage, the expression of SOX17 drops in the primitive gut tube and then is maintained during the formation of pancreatic progenitors suggesting that the CD275⁺ and CXCR4⁺ cells generate more ventral foregut endoderm cells compared to CD177⁺ cells (Fig. 3B). PDX1 expression is used as a control to assess differentiation of pancreatic progenitors. FACS data in Fig. 3E proves less expression of SOX17 in CD177⁺ cells compared to CD275⁺ and CXCR4⁺ cells.

To distinguish if the CD177⁺ cells generate more dorsal pancreatic progenitors, ventral (Fig. 3C) and dorsal pancreatic genes (Fig. 3D) were assessed using qPCR. Significantly higher levels of dorsal pancreatic markers such as Motor Neuron and Pancreas Homeobox 1 (MNX1), Patched 1 (PTCH1) and Noggin (NOG) were observed in CD177⁺ cells compared to CD275⁺ and CXCR4⁺ cells - indicating that CD177⁺ cells give rise to dorsal pancreatic progenitors while CD275⁺ and CXCR4⁺ cells give rise to ventral pancreatic progenitors.

### Reference Example 4: CD177⁺ subpopulations are specified pancreatic progenitors, while CD275⁺ subpopulations are liver primed progenitors

As confirmed in the last figure CD275⁺ and CXCR4⁺ cells give rise to ventral pancreatic progenitors while CD177⁺ cells give rise to dorsal pancreatic progenitors, it was decided to check for hepatic signatures in these subpopulations. For this, the cells were first differentiated towards DE, then sorted based on the antibodies and then these cells were further differentiated towards pancreatic progenitor stage using Rezania et al., 2014 protocol. An overview of the experimental setup is depicted in Fig. 4A.

After the differentiation towards pancreatic progenitor stage, total RNA was isolated and transcribed to cDNA for qPCR analysis. mRNA quantification showed that the liver progenitor marker genes such as HHEX, TTR and AFP were significantly upregulated in CD275⁺ and CXCR4+ cells compared to CD177⁺ (Fig. 4B) cells while pancreatic progenitor marker PDX1 was expressed more in CD177⁺ cells (Fig. 4B, C) making us believe that CD275⁺ and CXCR4⁺ cells give rise to ventral foregut endoderm and then ventral pancreas and liver while CD177⁺ cells give rise to dorsal foregut endoderm and dorsal pancreatic progenitors.

As liver cells are highly proliferative compared to pancreatic progenitors, which fall out of the cell cycle, we analysed the expression of β-catenin or WNT3A signature and the cell cycle profile of these specific subpopulations. WNT3A signalling pathway enhances the proliferation of the cells. This holds particularly true for the liver progenitors. Representative immunofluorescence stainings of β-catenin shows the translocation of β-catenin in CD177+ cells to the membrane while in CD275+ it is more localised in the nucleus and the cytoplasm (Fig. 4D). Representative FACS plots and graphical analysis of CD275⁺ and CD177⁺ cells stained for PI to show cell cycle status are shown in Fig. 4E. In general, it was observed that CD275+ cells that give rise to liver progenitors are highly proliferative subpopulation that has majority of cells in cycling S phase while the CD177+ cells that give rise to pancreatic progenitors fall out of cell cycle with the majority of cells in G1 phase (Fig. 4F), supporting our hypothesis.

### Reference Example 5: CD177⁺ DE generates compact, mature mono-hormonal beta-cells in vitro

As it is known that WNT/PCP pathway is activated in CD177⁺ subpopulation of DE, the differentiation potential of these cells towards pancreatic β-cells was further checked. It has already been proven that the activated WNT/PCP pathway aids in formation and maturation of pancreatic β-cells in vivo. Hence, the hESCs were differentiated towards DE, sorted using CD177 and CXCR4 (CD184) and then further differentiated towards pancreatic β-cells using Rezania et al., 2014 protocol. An overview of the experimental setup is depicted in Fig. 5A.

At stage 6, the cells were collected for mRNA quantification and FACS. For FACS, the cells were stained with pancreatic transcription factors PDX1 and NKX6.1. It was observed that CD177⁺ cells generated more PDX1⁺/NKX6.1⁺ cells compared to CXCR4+ cells (Fig. 5C). More interestingly, when stained for hormones insulin (INS) and glucagon (GLU or GCG), majority of CD177⁺ cells were INS⁺ while majority of CXCR4⁺ cells showed INS⁺/GLU⁺ indicating that the CXCR4⁺ cells give rise to majority of polyhormonal cells while CD177⁺ cells give rise to monohormonal β cells (Fig. 5B).

mRNA quantification of the pancreatic transcription genes such as PDX1, NKX6.1, NKX2.2 showed higher expression of these genes in CD177⁺ cells compared to CXCR4⁺ cells. CD177⁺ cells also showed higher expression of INS and lower levels of GCG genes compared to CXCR4⁺ cells. The most interesting fact of all is the higher expression of MAFA (MAF BZIP Transcription Factor A), a maturation marker in CD177⁺ β cells compared to CXCR4⁺ β cells. This expression of MAFA was comparable to its expression levels in human islets indicating that the activated WNT/PCP signalling in CD177⁺ cells can produce mature β-cells (Fig. 5D).

Another striking feature of sorting out CD177⁺ subpopulation is that it generated small compact structures compared to CXCR4⁺ cells. This compactness can be also attributed to activated non-canonical WNT signalling in CD177⁺ cells which can establish apical basal polarity in cells thereby aiding the maturity and self-organisation of CD177+ β cells (Fig. 5E).

### Reference Example 6: Further analysis of novel CD177+ and CD275+ ADE subpopulations

Self-organisation and spatial patterning has been recently studied using hESCs. Current hESC differentiation protocols induce an seemingly homogenous population of definitive endoderm (DE) when measured by one or two marker genes, i.e. CXCR4 or FOXA2/SOX17 at day 4 of differentiation (D4) (Fig. 6a-c). To investigate whether endoderm differentiation is homogenous the inventors used fluorescent activated cell sorting (FACS) based on CXCR4 (CD184) and c-KIT (CD117) marker expression and analyzed the induced endoderm by quantitative PCR (qPCR) (Fig. 6b and 6d-g). Surprisingly, the inventors found that CD117 marks only a subset of the CXCR4+ endoderm (63.3%) and that the CXCR4+/CD117+ double positive population can be divided into CXCR4+/CD117high (21.3%), CXCR4+/CD117mid (15.4%), CXCR4+/CD117low (26.6%) and CXCR4+/CD177- (15.3%) subpopulations (Fig. 6b). Interestingly, they found inverse expression levels of *FOXA2* and *SOX17* in the sorted CXCR4+/CD117high to CXCR4+/CD117low subpopulations (Fig. 6d, e) and increased expression of the ADE markers *CERBERUS1 (CER1)* and *HHEX* in certain subpopulations (Fig.6f, g). FoxA2 and Sox17 are expressed along opposite A-P gradients in the endoderm during mouse gastrulation. These results suggest that the endoderm is molecular heterogeneous and receives some kind of pattern information in culture through neighboring cell-type interactions, similar to the endoderm *in vivo.*

In XM001 hiPSC, H1 hESC and Mel1-NKX6.1-eGFP cell lines the CXCR4+ endoderm induction efficiency reaches 90% from which up to 1/3 CD275+ or 2/3 CD177+ ADE cells could be induced (Fig. 14A,B). For further analysis the hiPSC cell line was used due to its superior pancreatic differentiation capacity. When we compared three previously established endoderm induction schemes, the inventors observed profound differences in the induction efficiencies of the two ADE subpopulations at almost identical levels of CXCR4+ DE induction (Fig. 14C-G). Taken together, these results suggested that the inventors discovered two novel surface markers that identify distinct ADE subpopulations in the CXCR4+ bulk endoderm population. Of note, the ADE differentiation efficiency depends on chemical induction scheme and genetic background of the cell lines, implicating that quality control at early steps of differentiation is warranted to improve differentiation efficiencies, which can be done by use of the invention.

To further test whether ADE differentiation efficiency can be modulated by morphogens *in vitro* the inventors performed an inhibitor experiment where they induced endoderm by WNT3A and Activin A (AA) on the first day and then tuned BMP and/or TGF-β signalling strength during endoderm differentiation until D4 using the small molecule inhibitors DMH1 and SB431542, respectively (Fig. 14H). During standard differentiation, 20 ng/ml of WNT3A and 100 ng/ml AA induce 35.5% CD177+ ADE and 21% CD275+ ADE in the bulk CXCR4+ DE population (Fig.14I). High levels of TGF-β signalling (100 ng/ml AA) and BMP inhibition (4 µM DMH1) significantly increased CD177+ ADE from 35% to 80.5%, while CD275+ ADE induction was slightly decreased (14%) (Fig. 14J). On the other hand, low levels of BMP inhibitor (2.5 µM DMH1) and inhibition of TGF-β (1 µM SB431542) favoured CD275+ DE differentiation (54.0%) (Fig. 14K), suggesting that different quantities and qualities of ADE can be induced *in vitro.*

### Reference Example 7: CD177+ subpopulations differentiate into pancreatic progenitors and CD275+ subpopulations can be differentiated into liver progenitors.

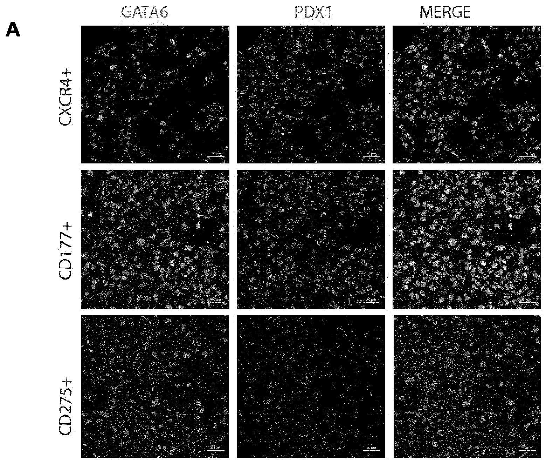

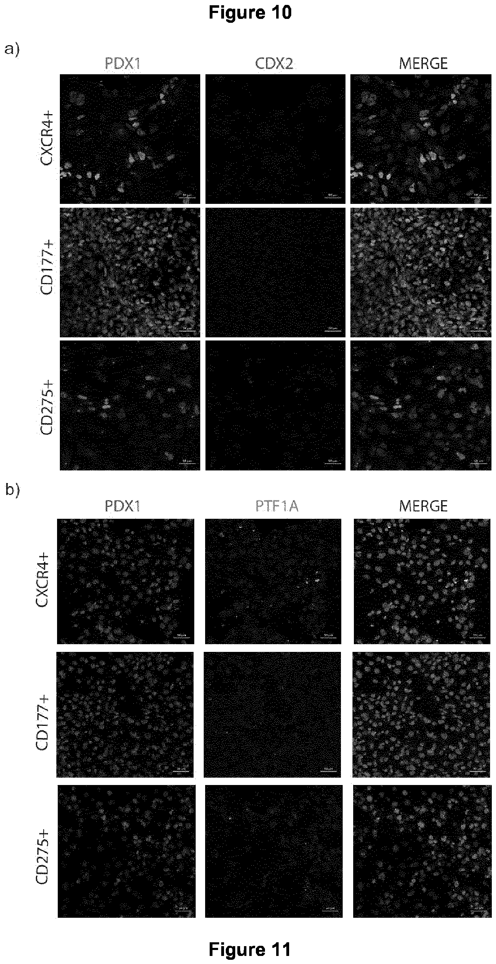

The inventors further studied in detail the surface marker expression of CXCR4, CD177 and CD275 during iPSC-derived pancreatic and endocrine differentiation *in vitro.* The expression of CD177 and CD275 peaked during ADE initiation and patterning and gradually decreased during gut tube and pancreatic progenitor formation, where the expression of CD177 and CD275 was almost negligible (Fig. 8E). However, the inventors observed high expression of CXCR4 during pancreatic and endocrine differentiation until the formation of iPSC derived β-like cells. To test whether the differential activation of cell fate specification pathways in CD177+ and CD275+ ADE subpopulations leads to preferential lineage allocation, the inventors explored the *in vitro* differentiation potential towards the liver and pancreatic fate. To test for liver potency the inventors differentiated the sorted cells towards hepatocytes33 and tested the expression of early liver progenitor genes *AFP, TTR* and *HHEX* (Fig. 8G). Remarkably, the expression of mRNA transcripts for *HHEX, AFP* and *TTR* were significantly upregulated in CXCR4+- and CD275+-derived early liver progenitors, when compared to CD177+-derived progenitors (Fig. 8G). On the other hand, when the inventors tested for pancreatic differentiation potency (Fig. 8A) they found around 68.8% of CD177+ ADE efficiently differentiated into PDX1+ pancreatic progenitors at PP1 stage, whereas only 50% of CXCR4+ - and 42.3% of CD275+-derived endoderm cells differentiate to the PP1 stage (Fig. 8A). Moreover, CD177+-derived PP1 cells expressed very high levels of PDX1 at PP1 stage (Fig. 8B, C). Immunofluorescence analysis substantiated these findings, suggesting that sorting of specified ADE subpopulations can enhance the differentiation potential towards the pancreatic fate (Fig. 8C, D). The inventors also analysed the expression of GATA6, an important regulator for pancreatic development in the enriched cells. CD177+-derived PPs showed more uniform expression of both GATA6 and PDX1, when compared to CXCR4+-and CD275+-derived PPs (Fig. 9A). To exclude the undirected generation of 10 other endoderm-derived organ progenitors we tested lung (SOX2+) and gut (CDX2+) markers, which were not expressed in any of the sorted subpopulations at PP2 stage (Fig. 10A). In addition, we ruled out pancreatic exocrine differentiation using PTF1A as a marker (Fig. 10B). Taken together, these results suggest that the CD177+ ADE subpopulation differentiates more uniform and efficiently into the pancreatic lineage.

### Reference Example 8: Inhibition of WNT3A secretion during gut tube patterning promotes the pancreatic fate

To test whether inhibition of WNT secretion promotes pancreatic fate induction, we enriched for CD275+, CD177+ and CXCR4+ subpopulations at DE stage and seeded them in 2D on Geltrex-coated dishes (Fig. 11A). The inventors used IWP2, a Porcupine chemical inhibitor that selectively inhibits palmitoylation and secretion of Wnt ligands. To check if the addition of IWP2 impacts the overall differentiation of ADE subpopulations towards the pancreatic fate, the inventors added IWP2 to sorted ADE subpopulations during primitive gut tube formation and patterning at S2 and then further differentiated towards PP2 at S4 (Fig. 11A). Under standard differentiation conditions without IWP2, FACS analysis for PDX1₊/NKX6.1₊ showed approx. 75% double positive PP2 cells derived from CD177+-enriched ADE, whereas only approx. 30% were generated from CD275+-derived ADE and approx. 45% from CXCR4+-derived DE (Fig. 11B, C). After the addition of IWP2, the percentages of PDX1+/NKX6.1+ PP2 cells derived from CXCR4+ (75%) and CD275+ (50%) increased drastically, suggesting that the inhibition of WNT secretion promotes pancreatic differentiation (Fig 11B, D). No differences were observed in the percentages of PDX1+/NKX6.1+ double positive PP2 cells in CD177+- derived PP2s, suggesting that CD177₊/CER1₊ ADE progenitors are shielded from canonical WNT signalling activation consistent with our previous results. Another hallmark of canonical Wnt/β-catenin pathway is the activation of cell-cycle regulators triggering cell proliferation. Cell proliferation assessment in CD177+-, CD275+- and CXCR4+-derived PPs by EdU pulse labelling revealed approx. 15% of CD177+-derived PPs were EdU+ comparedto 50% of CD275+- and 60% of CXCR4+-derived PPs (Fig. 11E, F). Suppression of NGN3 during the early stages of pancreatic differentiation is vital to reduce the generation of polyhormonal cells expressing insulin and glucagon39. Hence, we checked for the induction of NGN3 mRNA in CD177+-, CD275+- and CXCR4+-derived PP2s at S4 and in endocrine progenitors (EPs) at S5. NGN3 mRNA was tightly regulated in CD177+-derived PP2s and very efficiently induced in CD177+-derived EPs at S5, when compared to CXCR4+- and CD275+-derived PP2s and EPs in 2D cultures (Fig. 11G).

### Reference Example 9: CD177+ ADE generates efficiently SC-derived β-like cells

To determine whether CD177+-enriched ADE more efficiently generates SC-derived β-like cells the inventors differentiated MACS-enriched DE and ADE towards INS+/NKX6.1+ cells at S6 (Fig. 5A). Since CXCR4+-enriched DE and CD275+-enriched ADE expressed similar low mRNA levels of pancreatic and endocrine marker genes *PDX1, NKX6.1* and *NEUROD1* at S6 (Fig. 12A), the inventors decided to compare CD177+-enriched ADE to CXCR4+-enriched bulk DE. The mRNA transcripts of key β cell marker genes such as *PDX1, NKX6.1* and *NKX2.2,* were relatively higher in CD177+-derived β-like cells (Fig. 12A, G). Approximately, 65% of CD177+- and CXCR4+-derived β-like cells synthesized both PDX1 and NKX6.1 protein (Fig. 12B, D). Although, the protein levels of PDX1 and NKX6.1 were not different, the inventors noticed a striking difference in the mRNA transcripts of the hormones INS and *GCG* (Fig. 12H). Although comparable efficacies of PDX1 and NKX6.1 were observed between CXCR4+- and CD177+-derived β-like cells, the expression of C peptide +/PDX1+ and INS+/NKX6.1+ cells were reduced in CXCR4+-derived β-like cells (Fig. 12C-F). At S6, approx. 13.2% of CD177+-derived and 10.1% of CXCR4+-derived β-like cells were poly-hormonal cells (Fig. 12D, I).

### Reference Example 10: CD177+-enriched ADE generate more β-like cells in vitro

Subsequently, the inventors determined whether enrichment of ADE progenitors during early stages of differentiation improved differentiation, maturation and functionality. As the establishment of polarity and compaction plays an important role in maturation of β-like cells, the inventors decided to test if 2D vs 3D culture of the sorted cells influences the induction and expression of the key transcription factors PDX1 and NKX6.1 during pancreatic differentiation. Therefore, the inventors sorted CD177+ and CXCR4+ DE/ADE cells and further differentiated these into pancreatic progenitors either in 2D adherent cultures or after re-aggregation in 3D clusters (Fig. 13A). No evident difference was noted between the quantities of induced PDX1+/NKX6.1+ PP2s from the sorted and differentiated DE/ADE subpopulations, however, the inventors observed a slight increase in the amount of PDX1+/NKX6.1+ double positive PP2s in 3D culture (Fig. 13B). Hence, the inventors decided to differentiate the enriched DE/ADE subpopulations in 3D towards SC-derived β-like cells at S7. For this, the inventors enriched the CD177+- and CXCR4+-endoderm populations and plated the cells on low attachment dishes to re-aggregate and form 3D clusters. Differentiation to S7 was carried out on aggregated 3D clusters based on our modified protocol (Fig. 13C). Interestingly, it was observed that the formation of dense and compact aggregates with defined ECAD+ adherence junctions in CD177+-derived β-like 3D clusters (Fig. 13D) in line with the enrichment of β-catenin in CD177+ ADE progenitors (Fig. 4D). First, it was confirmed that CD177+-derived β-like cells expressed the mature β cell transcription factor MAFA. Almost 60% of CD177+-derived β-like cells expressed INS and MAFA (Fig. 13F, H, J). In CD177+-derived β-like cells, the inventors also found elevated levels of *MAFA, GLUT1* and *UCN3* mRNA transcripts (Fig. 13E). Notably, the mRNA expression of glucose transporter *GLUT1* and *UCN3* was significantly higher in CD177+- compared to CXCR4+-derived β-like cells, implicating that the isolation of pancreatic specified ADE promotes more homogenous differentiation towards β-like cells when compared to heterogeneous CXCR4+ bulk DE (Fig. 13Ee). Protein quantification by FACS confirmed approx. 70% of CD177+-derived β-like cells expressed GLUT1 and INS (Fig. 13F, K). Immunocytochemistry data proved the presence of GLUT1 in the membrane of CD177-derived β-like cells, while very low expression of GLUT1 was noticed in CXCR4+-derived β-like cells signifying improved maturation and functional state of CD177-derived β-like cells (Fig. 13F, J). Finally, we tested the functionality of CD177+- and CXCR4+-derived β-like cells by static glucose stimulated insulin secretion (GSIS). CD177+-derived β-like cells exhibited a clearly improved response upon 1 h static glucose stimulation with 16.7 mM glucose (Fig. 13G).

## Claims

1. A method of purifying a pancreatic progenitor cell comprising:
(a) exposing a population of cells derived from pluripotent stem cells, induced pluripotent stem cells or embryonic stem cells comprising a pancreatic progenitor cell, to a ligand which binds to a cell surface marker on the pancreatic progenitor cell, wherein the cell surface marker is CD177 and
(b) separating the pancreatic progenitor cell from cells derived from pluripotent stem cells, induced pluripotent stem cells or embryonic stem cells which do not bind to the ligand, thereby purifying said pancreatic progenitor cell,
wherein the pancreatic progenitor cell is sorted at the stage of the definitive endoderm (DE), the anterior definitive endoderm (ADE), the posterior gut tube (PGT) and/or the ventral foregut (VFG).

2. The method of claim 1, wherein the pancreatic progenitor cells express FOXA2, SOX17, CER1 and/or CXCR4, preferably CER1.

3. The method of claim 1 or 2, further comprising the step exposing said population of cells to a ligand which binds to CD51 and/or CD275.

4. The method of any one of claims 1 to 3,
wherein the ligand is an antibody or binding fragment thereof, preferably wherein said antibody is a monoclonal antibody or a polyclonal antibody; or
wherein the ligand is conjugated with a label.

5. The method of any one of the preceding claims,
wherein the expression of the marker is detected by quantitative polymerase chain reaction (qPCR); and/or
wherein the expression of the marker is detected by flow cytometry.

6. The method of any one of the preceding claims,
wherein the separating step comprises affinity chromatography or wherein the separating step comprises flow cytometry.

7. The method of any one of claims 1 to 6, wherein the pancreatic progenitor cells are human.

8. A purified composition comprising at least 50% pancreatic progenitor cells which are positive for CD177.

9. The composition of claim 8, wherein the pancreatic progenitor cells are bound to a ligand that binds CD177 on the pancreatic progenitor cells.

10. The composition of claim 8 or 9 wherein the pancreatic progenitor cells are human.

11. The method of any one of claims 1 to 6, or the composition of any one of claims 8 to 10,
wherein the ligand is immobilized on a capture medium, preferably wherein the capture medium comprises a bead; or
wherein the ligand is conjugated to a magnetic reagent.

12. The method of any one of claims 1 to 7, wherein the purified cell composition obtainable by the method comprises at least 50% pancreatic progenitor cells.

13. Use of a ligand which binds to a cell surface marker on a pancreatic progenitor cell, wherein the cell surface marker is CD177, for purifying a pancreatic progenitor cell from a population of cells derived from pluripotent stem cells, induced pluripotent stem cells or embryonic stem cells, wherein the pancreatic progenitor cell is purified at the stage of the definitive endoderm (DE), the anterior definitive endoderm (ADE), the posterior gut tube (PGT) and/or the ventral foregut (VFG).

## Patentansprüche

1. Verfahren zum Aufreinigen einer Vorläuferzelle der Bauchspeicheldrüse, umfassend:
(a) Aussetzen einer Population von Zellen, die von pluripotenten Stammzellen, induzierten pluripotenten Stammzellen oder embryonalen Stammzellen abgeleitet sind, umfassend eine Vorläuferzelle der Bauchspeicheldrüse, einem Liganden, der an einen Zelloberflächenmarker auf der Vorläuferzelle der Bauchspeicheldrüse bindet, wobei der Zelloberflächenmarker CD177 ist und
(b) Trennen der Vorläuferzelle der Bauchspeicheldrüse von Zellen, die von pluripotenten Stammzellen, induzierten pluripotenten Stammzellen oder embryonalen Stammzellen abgeleitet sind, die nicht an den Liganden binden, wodurch die Vorläuferzelle der Bauchspeicheldrüse aufgereinigt wird,
wobei die Vorläuferzelle der Bauchspeicheldrüse im Stadium des definitiven Endoderms (DE), des anterioren definitiven Endoderms (ADE), des posterioren Darmrohrs (PGT) und/oder des ventralen Vorderdarms (VFG) sortiert wird.

2. Verfahren nach Anspruch 1, wobei die Vorläuferzellen der Bauchspeicheldrüse FOXA2, SOX17, CER1 und/oder CXCR4, vorzugsweise CER1, exprimieren.

3. Verfahren nach Anspruch 1 oder 2, ferner umfassend den Schritt des Aussetzens der Population von Zellen einem Liganden, der an CD51 und/oder CD275 bindet.

4. Verfahren nach einem der Ansprüche 1 bis 3,
wobei der Ligand ein Antikörper oder ein Bindungsfragment davon ist, vorzugsweise wobei der Antikörper ein monoklonaler Antikörper oder ein polyklonaler Antikörper ist; oder
wobei der Ligand mit einem Label konjugiert ist.

5. Verfahren nach einem der vorhergehenden Ansprüche,
wobei die Expression des Markers durch quantitative Polymerase-Kettenreaktion (qPCR) nachgewiesen wird; und/oder
wobei die Expression des Markers durch Durchflusszytometrie nachgewiesen wird.

6. Verfahren nach einem der vorhergehenden Ansprüche,
wobei der Trennschritt Affinitätschromatographie umfasst oder wobei der Trennschritt Durchflusszytometrie umfasst.

7. Verfahren nach einem der Ansprüche 1 bis 6, wobei die Vorläuferzellen der Bauchspeicheldrüse menschlich sind.

8. Aufgereinigte Zusammensetzung umfassend mindestens 50% Vorläuferzellen der Bauchspeicheldrüse, die für CD177 positiv sind.

9. Zusammensetzung nach Anspruch 8, wobei die Vorläuferzellen der Bauchspeicheldrüse an einen Liganden gebunden sind, der CD177 auf den Vorläuferzellen der Bauchspeicheldrüse bindet.

10. Zusammensetzung nach Anspruch 8 oder 9, wobei die Vorläuferzellen der Bauchspeicheldrüse menschlich sind.

11. Verfahren nach einem der Ansprüche 1 bis 6, oder die Zusammensetzung nach einem der Ansprüche 8 bis 10,
wobei der Ligand auf einem Aufnahmemedium immobilisiert ist, vorzugsweise wobei das Aufnahmemedium einen Bead umfasst; oder
wobei der Ligand an ein magnetisches Reagenz konjugiert ist.

12. Verfahren nach einem der Ansprüche 1 bis 7, wobei die aufgereinigte Zellzusammensetzung erhältlich durch das Verfahren mindestens 50% Vorläuferzellen der Bauchspeicheldrüse umfasst.

13. Verwendung eines Liganden, der an einen Zelloberflächenmarker auf einer Vorläuferzelle der Bauchspeicheldrüse bindet, wobei der Zelloberflächenmarker CD177 ist, zum Aufreinigen einer Vorläuferzelle der Bauchspeicheldrüse aus einer Population von Zellen, die von pluripotenten Stammzellen, induzierten pluripotenten Stammzellen oder embryonalen Stammzellen abgeleitet sind, wobei die Vorläuferzelle der Bauchspeicheldrüse im Stadium des definitiven Endoderms (DE), des anterioren definitiven Endoderms (ADE), des posterioren Darmrohrs (PGT) und/oder des ventralen Vorderdarms (VFG) aufgereinigt wird.

## Revendications

1. Procédé de purification d'une cellule progénitrice pancréatique comprenant :
(a) l'exposition d'une population de cellules dérivées de cellules souches pluripotentes, de cellules souches pluripotentes induites ou de cellules souches embryonnaires comprenant une cellule progénitrice pancréatique, à un ligand qui se lie à un marqueur de surface cellulaire sur la cellule progénitrice pancréatique, le marqueur de surface cellulaire étant CD177, et
(b) la séparation de la cellule progénitrice pancréatique des cellules dérivées de cellules souches pluripotentes, cellules souches pluripotentes induites ou cellules souches embryonnaires qui ne se lient pas au ligand, purifiant ainsi ladite cellule progénitrice pancréatique,
dans lequel la cellule progénitrice pancréatique est triée au stade de l'endoderme définitif (DE), de l'endoderme définitif antérieur (ADE), du tube digestif postérieur (PGT) et/ou de l'intestin antérieur ventral (VFG).

2. Procédé selon la revendication 1, dans lequel les cellules progénitrices pancréatiques expriment FOXA2, SOX17, CER1 et/ou CXCR4, de préférence CER1.

3. Procédé selon la revendication 1 ou 2, comprenant en outre l'étape d'exposition de ladite population de cellules à un ligand qui se lie à CD51 et/ou CD275.

4. Procédé selon l'une quelconque des revendications 1 à 3,
dans lequel le ligand est un anticorps ou un fragment de liaison de celui-ci, de préférence dans lequel ledit anticorps est un anticorps monoclonal ou un anticorps polyclonal ; ou
dans lequel le ligand est conjugué à une étiquette.

5. Procédé selon l'une quelconque des revendications précédentes,
dans lequel l'expression du marqueur est détectée par une réaction en chaîne par polymérase quantitative (qPCR) ; et/ou
dans lequel l'expression du marqueur est détectée par cytométrie en flux.

6. Procédé selon l'une quelconque des revendications précédentes,
dans lequel l'étape de séparation comprend la chromatographie d'affinité ou dans lequel l'étape de séparation comprend la cytométrie en flux.

7. Procédé selon l'une quelconque des revendications 1 à 6, dans lequel les cellules progénitrices pancréatiques sont humaines.

8. Composition purifiée comprenant au moins 50 % de cellules progénitrices pancréatiques qui sont positives pour CD177.

9. Composition selon la revendication 8, dans laquelle les cellules progénitrices pancréatiques sont liées à un ligand qui lie CD177 sur les cellules progénitrices pancréatiques.

10. Composition selon la revendication 8 ou 9, dans laquelle les cellules progénitrices pancréatiques sont humaines.

11. Procédé selon l'une quelconque des revendications 1 à 6, ou composition selon l'une quelconque des revendications 8 à 10, dans lequel ou dans laquelle le ligand est immobilisé sur un milieu de capture, de préférence dans lequel ou dans laquelle le milieu de capture comprend une bille ; ou
dans lequel ou dans laquelle le ligand est conjugué à un réactif magnétique.

12. Procédé selon l'une quelconque des revendications 1 à 7, dans lequel la composition de cellules purifiée pouvant être obtenue par le procédé comprend au moins 50 % de cellules progénitrices pancréatiques.

13. Utilisation d'un ligand qui se lie à un marqueur de surface cellulaire sur une cellule progénitrice pancréatique, dans laquelle le marqueur de surface cellulaire est CD177, pour purifier une cellule progénitrice pancréatique à partir d'une population de cellules dérivées de cellules souches pluripotentes, de cellules souches pluripotentes induites ou de cellules souches embryonnaires, la cellule progénitrice pancréatique étant purifiée au stade de l'endoderme définitif (DE), de l'endoderme définitif antérieur (ADE), du tube digestif postérieur (PGT) et/ou de l'intestin antérieur ventral (VFG).
